# EUROPEAN PATENT APPLICATION

(11) **EP 1 323 735 A1**
(43) Date of publication of application: **02.07.2003**
(21) Application number: 01967724.4
(22) Date of filing: 18.09.2001
(51) Int. Cl.: C07K 14/47, C12N 15/09, C07K 16/18, C12N 1/15, C12N 1/19, C12N 1/21, C12N 5/10, C12P 21/02, C12P 21/08, A61K 45/00, A61K 31/711, A61P 43/00, A61P 35/00

(54) **GENE RELATING TO DRUG TOLERANCE AND UTILIZATION THEREOF**

(30) Priority: 03.10.2000 JP 2000303441
(71) Applicant: BANYU PHARMACEUTICAL CO., LTD., Chuo-ku, Tokyo 103-8416 (JP)
(72) Inventor: KOMATANI, H., c/o BANYU PHARMACEUTICAL CO., LTD., Tsukuba-shi, Ibaraki 300-2611 (JP); HARA, Y., c/o BANYU PHARMACEUTICAL CO., LTD., Tsukuba-shi, Ibaraki 300-2611 (JP); KOTANI, H., c/o BANYU PHARMACEUTICAL CO., LTD., Tsukuba-shi, Ibaraki 300-2611 (JP); NAKAGAWA, R., c/o BANYU PHARMACEUTICAL CO., LTD., Tsukuba-shi, Ibaraki 300-2611 (JP)
(74) Representative: Nash, David Allan
(86) International application number: JP0108112
(87) International publication number: WO02028894

(57) **Abstract**

By using an anticancer drug having a unique structure which has never been reported as being transported by a known transporter protein capable of conferring resistance on a cancer cell, cell lines resistant to this anti-cancer drug are established. Then a cDNA encoding a protein which is expressed at a high level in these resistant cell lines is successfully isolated. Use of these gene and protein makes it possible to detect cell lines resistant to this anticancer drugs, and select an anticancer drug which can be administered to a cancer patient. Moreover, a candidate compound for an inhibitor of the transporter can be screened thereby, and the sensitivity of a cancer cell resistant to the anticancer drug can be enhanced by using a combination of the inhibitor thus obtained with the anticancer drug.

## Description

### Technical Field

The present invention is useful in a field of pharmaceutical products. More specifically, the present invention relates to a novel protein which excretes cancer chemotherapeutic drugs from a cell and which confers drug-resistance on the cell, a DNA encoding the protein, a method for detecting a cell type resistant to cancer chemotherapeutic drugs, indication for selecting a cancer chemotherapeutic drug for a patient, and a method for screening an inhibitor to overcome the resistance to cancer chemotherapeutic drugs.

### Background Art

The development of resistance to cancer chemotherapeutic drugs is a major problem in the treatment of cancer by conventional cytotoxic drugs. Tumors are sensitive to chemotherapeutic drugs at first, however, exhibit multidrug resistance afterward, and result in relapse. As a major cause for such chemotherapeutic drug resistance, there is a decrease of intracellular drug concentration by excretion of the drug. This mechanism depends on the expression of a transporter that excretes chemotherapeutic drugs out of the cancer cell. P-glycoprotein encoded on *MDR1* gene (hereinafter referred to as "P-gp") and a multidrug resistance-associated protein encoded on *MRP* gene (hereinafter referred to as "MRP") have been reported as typical members of such transporters. P-gp is a molecular pump which has been previously known to be involved in multidrug resistance to multiple types of tumor, and MRP is a transporter that was shown to be involved in multidrug resistance to lung cancer at first and then revealed to be expressed in other types of cancer as well (Cole, S. P.C. *et al*., *Science* 258, 1650-1654 (1992)) (Slovak, M. L. *et al., Cancer Res.* 53, 3221-3225 (1993)).

Each of these proteins is a member of ABC (ATP-binding cassette) transporter superfamily, and a molecule that is localized in a cell membrane and transports its substrates utilizing an energy source such as ATP hydrolysis.

Recently, various new ABC family molecules have been discovered in succession, and these molecular pumps that are suggested to be involved in drug resistance in addition to P-gp and MRP, have been studied. Some of the molecules make up a sub-family referred to as ABCG2 (BCRP/MXR/ABCP). The genes for this sub-family were reported as *ABCP* that was specifically expressed in placenta (Allikmets, R. *et al*., *Cancer Res.* 58, 5337-5339 (1998)), *BCRP* that was isolated from a resistant cell line selected with adriamycin (Doyle, A. *et al*., *Proc. Natl. Acad. Sci. U.S.A.* 95, 15665-15670 (1998)), and *MXR* that was isolated from a resistant cell line selected with mitoxantrone (Miyake, K. *et al., Cancer Res.* 59, 8-13 (1999)). In these three kinds of genes, variations of 1 to 4 amino acid sequences derived from the nucleotide substitutions between the respective genes were observed.

From the analysis of the cell line which is produced by introducing and expressing the nucleotide sequence reported as *BCRP* into MCF-7 cell, expression of this gene was shown to confer resistance to mitoxantrone and adriamycin. Thus, the gene has been notable for a novel factor of multidrug resistance (Doyle, A. *et al*., Proc. Natl. Acad. Sci. U.S.A. 95, 15665-15670 (1998) and WO 99/40110).

In the above reports, however, there is only disclosed the relationship between cancer chemotherapeutic drugs having an anthraquinone skeleton such as adriamycin, daunorubicin and mitoxantrone and the above-mentioned genes. In addition, there is no disclosure or suggestion concerning the relationship between amino acid replacement of the genes and the substrate specificity.

Recently, it was reported that the expression of ABCG2 (BCRP /MXR/ABCP) sub-family might be correlated with the resistance to topotecan that do not have an anthraquinone skeleton (Maliepaard, M. *et al*., *Cancer Res.* 59, 4559-4563 (1999)), however, it was not proved distinctly.

On the other hand, with respect to the drug resistant mechanisms to the compounds represented by the following general formula (I) (hereinafter referred to as "indolocarbazole compound"): 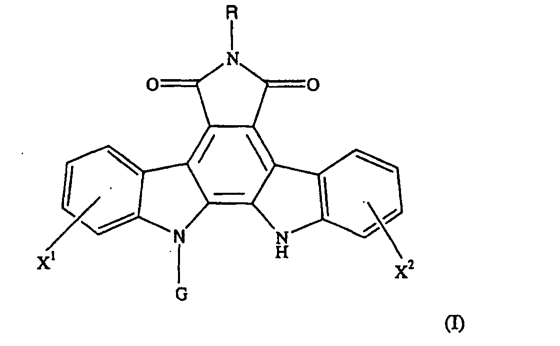 wherein X¹ and X² each independently represent a hydrogen atom, halogen atom or hydroxyl group; R represents a hydrogen atom, amino, formylamino, or lower alkylamino which may be substituted with any one selected from the group consisting of one to three hydroxyl group(s), a pyridyl group optionally having substituent(s), and thienyl group optionally having substituent(s); and G represents a pentose group, hexose group or derivative thereof, which may be substituted with an amino group, it has been reported that the growth inhibitory effect of Compound-A (in the general formula (I), X¹ represents 1-hydroxyl group, X² represents 11-hydroxyl group, R represents a formylamino and G represents β-D-glucopyranosyl group) to various cells was correlated with the accumulation of the Compound-A in the cells (Yoshinari, T. *et al*., *Cancer Res*. 55, 1310-1315 (1995) and Kanzawa, F. *et al*., *Cancer Res.* 55, 2806-2813 (1995)).

In the above report, however, it was only disclosed that the resistance caused by the change of intracellular accumulation of the drug was not correlated with the function of the P-gp. Including all above reports, there is no report which has revealed a factor concerning the intracellular accumulation or drug resistance to the indolocarbazole compound in the past.

### Problem to be solved by the Invention

With respect to cancer chemotherapeutic drugs having an anthraquinone skeleton (adriamycin, doxorubicin, mitoxantrone and the like), the P-gp, MRP and BCRP are thought to be involved in resistance to the drugs. Therefore, these are not desirable cancer chemotherapeutic drugs. In view of such situations, it is desired to develop anti-cancer drugs that are not transported by P-gp and the like.

The above-mentioned compounds having an indolocarbazole skeleton were not reported to be involved in resistance by the P-gp, MRP and BCRP, so Compound-A (Yoshinari, T. *et al., Cancer Res.* 55, 1310-1315 (1995)), and Compound-B (in the above general formula (I), X¹ represents 2-hydroxyl group, X² represents 10-hydroxyl group, R represents (1-hydroxymethyl-2-hydroxyl) ethylamino group, and G represents β-D-glucopyranosyl group) (Yoshinari, T. *et al., Cancer Res.* 59, 4271-4275 (1999)) both of which are topoisomerase I inhibitors, and UCN-01 which is a protein kinase inhibitor (Akinaga, S. *et al., Cancer Res.* 51, 4888-4892 (1991)) promise to be novel anticancer candidates. However, it is important to clarify a factor involved in the change of intracellular accumulation of indolocarbazole compounds in view of the above disclosure of Yoshinari *et al*.

Accordingly, it is an object of the present invention to provide a protein which is relevant to the intracellular accumulation of indolocarbazole compounds and a polynucleotide coding therefor. It is also an object of the present invention to provide a method for detecting an expression of the gene in a cancer cell or cancer patient using the polynucleotide coding for the protein or an antibody against the protein. It is further an object of the present invention to provide a method for screening an inhibitor to overcome the resistance to cancer chemotherapeutic drugs using the gene and the protein.

### Disclosure of the Invention

The present inventors have been studied hard to solve the above problems. Firstly, they established cell lines resistant to Compound-A. More specifically, these cell lines were obtained by cultivating mouse cell line LY, human cell line HCT116, and human cell line PC-13 under the presence of Compound-A for a long time. All of the obtained Compound-A resistant cell lines, i.e., LY/NR2, HCT116/NR1, and PC-13/NR13, and a spontaneous cell line HeLa #7 showed 50 to several thousand fold resistance to indolocarbazole compounds such as Compound-A, whereas only 20 fold or the like to other anticancer drugs such as camptothecin, adriamycin and mitoxantrone. Thus, the existence of a resistant mechanism specific to indolocarbazole compounds in these resistant cell lines was suggested. It was also speculated that a factor exists which decreases the intracellular concentration of indolocarbazole compounds and causes the resistance to the compounds in the resistant cell lines by finding decreased intracellular accumulation of indolocarbazole compounds such as Compound-A in the resistant cell lines.

Next, the present inventors compared the expressions of total known transcripts between the mouse cell line LY/NR2 resistant to Compound-A and the parent cell line LY by using DNA chips. As a result, a genomic sequence (*ABCG2* of the present invention) having a high homology with the gene of ABCG2 sub-family which is a member of ABC transporter was found as a gene which showed the most increased expression level specific to the resistant cell line.

According to Northern analysis, it was found that the above gene was overexpressed not only in the mouse cell line LY/NR2, but also in all of the obtained human resistant cell lines. Therefore, in view of the fact that the *ABCG2* gene of the present invention was expressed in all of the cell lines resistant to Compound-A and the previous finding that the aforementioned *BCRP* and the like are associated with the excretion of anti-cancer drugs, it was strongly suggested that the gene product of the present invention was a factor that confers the resistance to indolocarbazole compounds.

Then, the present inventors isolated a full-length cDNA of the present invention from human spontaneous resistant cell line HeLa #7 by RT-PCR method using primers designed based on the previous human *ABCG2* sequence, determined the nucleotide sequence, and have found that the sequence of gene of the present invention was a novel one different from any other *ABCG2* sequences previously reported. Namely, the gene of the present invention was a variant in which the codon sequence coding for threonine at amino acid residue 482 of the amino acid sequence reported as BCRP was changed to that coding for arginine by a single nucleotide substitution. Further, the present inventors determined the nucleotide sequence of *ABCG2* which is obtained from cDNA derived from normal human tissues, i.e. placenta and kidney, wherein they focused attention on the part of the sequence different from the existing *ABCG2,* i.e. *BCRP* and *MXR*, sequences and have found out that the protein that has the same nucleotide sequence as that obtained from HeLa #7 cell line was expressed in normal human tissues.

The present inventors studied whether the gene of the present invention was capable of conferring resistance to indolocarbazole compounds on a cell. For the sake of study, a full length gene isolated from HeLa#7 was ligated with an expression vector and a stably transformed PC-13 cell line in which the gene was forcedly expressed was produced. As a result, the cell in which the *ABCG2* gene of the present invention was forcedly expressed was approximately 10 to 20 fold resistant to indolocarbazole compounds. It was therefore proved that the gene of the present invention is a resistant factor to indolocarbazole compounds. At the same time, the present inventors have found that the cell expressing the gene of the present invention was not resistant to mitoxantrone and adriamycin in contrast to the previously reported cell line expressing the BCRP. Thus, it was proved that the gene of the present invention encoding a protein which has one amino acid replacement is a new type of gene having a novel activity.

Further, in order to clarify the relationship between one amino acid difference observed in the sequences of ABCG2 of the present invention compared with the sequence reported as BCRP (Doyle, A. *et al., Proc. Natl. Acad. Sci. U.S.A.* 95, 15665-15670 (1998) and WO99/40110) and the resistance to each drug, the present inventors introduced each of *ABCG2* of the present invention and an amino acid variant of *ABCG2* (*ABCG2-482T*) in which the codon of the 482nd amino acid was replaced to that of threonine as reported for *BCRP*, into MCF-7 cell and compared the characteristics of the cells.

As a result, the cell in which *ABCG2-482T* was introduced showed resistance to mitoxantrone and adriamycin in addition to Compound-B as an indolocarbazole compound, whereas the cell in which *ABCG2* of the present invention was introduced showed strong resistance only to Compound-B. Thus, it was proved that the *ABCG2* gene of the present invention shows the selective resistance to indolocarbazole compounds. There was also observed the same selectivity difference with respect to the accumulation of these compounds in the cells, and it was suggested that the selective resistance to indolocarbazole compounds cuased by ABCG2 of the present invention is due to the substrate specific transport by this molecule. As a consequence of these observations, the distinct difference between the characteristics of ABCG2 as was previously reported BCRP and the ABCG2 of the present invention has been found out and it was shown that the difference was based on one amino acid substitution at codon 482.

Accordingly, in view of the above evidence, i.e. the fact that the *ABCG2* gene of the present invention confers the resistance selective to indolocarbazole compounds on a cell, the present inventors have found that the gene and the protein of the present invention and fragments thereof or the antibodies thereagainst are applicable to detect the gene expression in a cancer patient and predict whether the cancer patient is resistant selective to indolocarbazole compounds or not. It was further found that the screening of compounds which regulates the expression of the gene or the activity of the gene product can be developed, and the application of the compounds to overcome the drug resistance.

Namely, the present invention relates to the human ABCG2 protein (hereinafter referred to as "ABCG2 of the present invention") that confers the resistance to cancer chemotherapeutic drugs on a mammalian cell, and the protein coding therefor as well as the production and use thereof. More specifically, the present invention relates to:
(1) A protein consisting of an amino acid sequence of the following (a) or (b), and conferring the resistance to a cancer chemotherapeutic drug(s) on a mammalian cell:
   (a) an amino acid sequence of SEQ ID NO:2,
   (b) an amino acid sequence of SEQ ID NO:2, wherein one or several amino acids are replaced, deleted or added.
(2) The protein of (1), wherein said cancer chemotherapeutic drug(s) is/are a compound(s) represented by the following general formula (I): 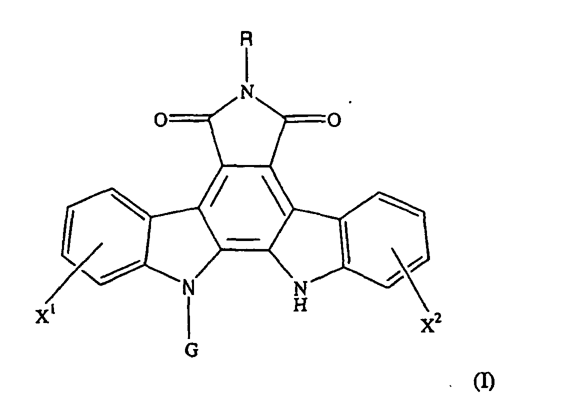
wherein X¹ and X² each independently represent a hydrogen atom, halogen atom or hydroxyl group;
R represents a hydrogen atom, amino, formylamino, or lower alkylamino which may be substituted with any one selected from the group consisting of one to three hydroxyl group(s), a pyridyl group optionally having substituent(s), and thienyl group optionally having substituent(s); and
G represents a pentose group, hexose group or derivative thereof which may be substituted with an amino group.
(3) A partial peptide of the protein of (1) or (2).
(4) A polynucleotide which encodes the protein or the partial peptide of any one of (1) to (3), or a complementary polynucleotide thereto.
(5) The polynucleotide of (4), wherein the DNA sequence of said polynucleotide consists of at least a part of the coding region of SEQ ID NO:1 or the complementary sequence thereto.
(6) The polynucleotide of (4) or (5), which is used for detecting a mammalian cell having the resistance to a compound represented by the following general formula (I): 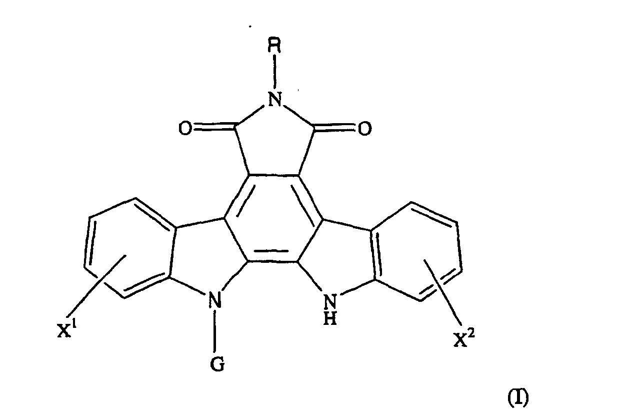
wherein X¹ and X² each independently represent a hydrogen atom, halogen atom or hydroxyl group;
R represents a hydrogen atom, amino, formylamino, or lower alkylamino which may be substituted with any one selected from the group consisting of one to three hydroxyl group(s), a pyridyl group optionally having substituent(s), and thienyl group optionally having substituent(s); and
G represents a pentose group, hexose group or derivative thereof which may be substituted with an amino group.
(7) A polynucleotide consisting of the contiguous 15 to 100 DNA sequence of SEQ ID NO:1, said sequence comprising a G at nucleotide number 1489 of DNA sequence represented in SEQ ID NO:1, or a complementary polynucleotide thereto.
(8) A recombinant vector comprising the polynucleotide of (4) or (5).
(9) A transformant comprising the vector of (8).
(10) A method for producing the protein or the partial peptide of any one of (1) to (3) , comprising the steps of:
   cultivating the transformant of (9), and
   recovering the expressed protein or the partial peptide from said transformant or the supernatant thereof.
(11) An antibody which specifically binds to the protein or the partial peptide of any one of (1) to (3).
(12) The antibody of (11), which is used for detecting a mammalian cell having the resistance to a cancer chemotherapeutic drug(s) represented by the following general formula (I): 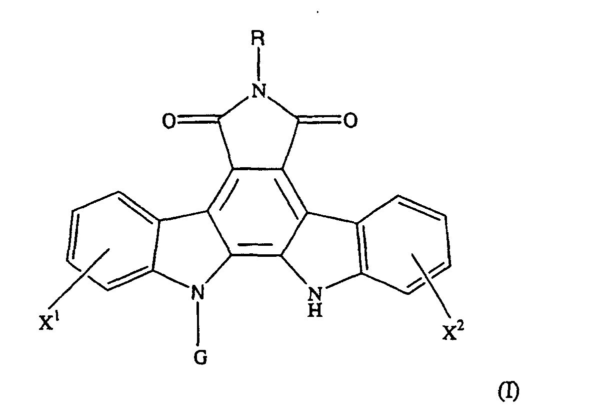
wherein X¹ and X² each independently represent a hydrogen atom, halogen atom or hydroxyl group;
R represents a hydrogen atom, amino, formylamino, or lower alkylamino which may be substituted with any one selected from the group consisting of one to three hydroxyl group(s), a pyridyl group optionally having substituent(s), and thienyl group optionally having substituent(s); and
G represents a pentose group, hexose group or derivative thereof which may be substituted with an amino group.
(13) An antisense nucleotide(s) which suppresses the expression of the protein of (1) or (2).
(14) A method for predicting the resistance to a cancer chemotherapeutic drug of a patient, said resistance being indicated by the expression of the protein of (1) or (2).
(15) The method of (14), wherein said cancer chemotherapeutic drug is a compound represented by the following general formula (I): 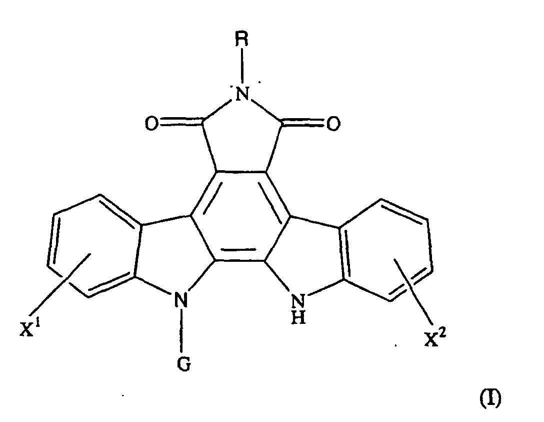
wherein X¹ and X² each independently represent a hydrogen atom, halogen atom or hydroxyl group;
R represents a hydrogen atom, amino, formylamino, or lower alkylamino which may be substituted with any one selected from the group consisting of one to three hydroxyl group(s), a pyridyl group optionally having substituent(s), and thienyl group optionally having substituent(s); and
G represents a pentose group, hexose group or derivative thereof which may be substituted with an amino group.
(16) A method for screening a compound which inhibits the function of the protein of (1) or (2), comprising:
   (a) making said protein, a cancer chemotherapeutic drug and a candidate compound in contact with one another, and
   (b) selecting the candidate compound as a desired candidate compound in case where the candidate compound suppresses the activity of said protein.
(17) The method of (16), wherein the activity of said protein, (one of) whose substrate is said cancer chemotherapeutic drug, consists of a binding activity with said substrate , ATP degrading activity when said substrate binds thereto, or a transporting activity of said substrate.
(18) A method for screening a compound which inhibits the function of the protein of (1) or (2), comprising:
   (a) contacting a mammalian cell that expresses the protein of claim 1 or 2 with a cancer chemotherapeutic drug and a candidate compound, and
   (b) selecting the candidate compound as a desired candidate compound in case where the candidate compound enhances the toxicity of said cancer chemotherapeutic drug against said mammalian cell.
(19) An inhibitor obtained by any one of methods (16) to (18).
(20) A method for inhibiting the function of the protein of (1) by using the inhibitor of (19).
(21) A method for inhibiting the function of the protein of (1) by using the antibody of (11) or (12).
(22) A method for inhibiting the function of the protein of (1) by using the antisense nucleotide(s) of (13).
(23) A method for improving the sensitivity of a patient to a cancer chemotherapeutic drug, said method comprising any one of methods (20) to
(22) in order to inhibit the function of the protein of (1).
(24) The method of (23), wherein said cancer chemotherapeutic drug is a compound represented by the following general formula (I): 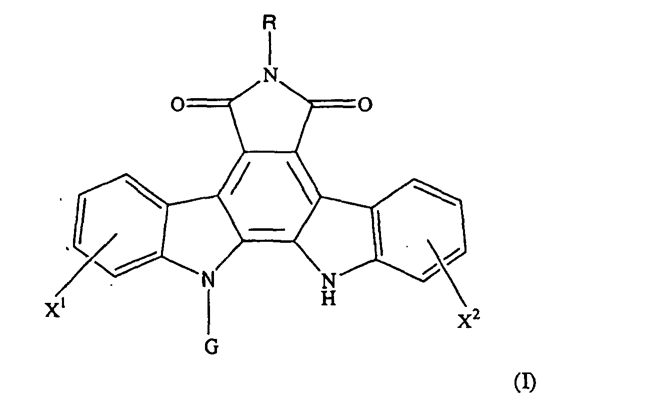
wherein X¹ and X² each independently represent a hydrogen atom, halogen atom or hydroxyl group;
R represents a hydrogen atom, amino, formylamino, or lower alkylamino which may be substituted with any one selected from the group consisting of one to three hydroxyl group(s), a pyridyl group optionally having substituent(s), and thienyl group optionally having substituent(s); and
G represents a pentose group, hexose group or derivative thereof which may be substituted with an amino group.

### Brief Description of the Drawings

Fig.1 shows the expression of transcripts of the *ABCG2* gene of the present application in Compound-A resistant cell lines.
Fig.2 shows the expression of *ABCG2* transcripts in PC-13 cell lines to which the *ABCG2* gene of the present application was introduced.
Fig.3 shows the accumulation of Compound-B in PC-13 cell lines to which the *ABCG2* gene of the present application was introduced.
Fig.4 shows the method of introducing an amino acid substitution in the *ABCG2* gene using a two-step PCR: (a) a primer design at a base substitution site, (b) outline of the two-step PCR.
Fig.5 shows the expression of each of *ABCG2* transcripts in MCF-7 cells to which the *ABCG2* gene of the present application or *ABCG2-482T* gene was introduced. In the drawing, "vector" represents MCF-7 cell with introduced the vector alone, "T8" represents MCF-7 cell with introduced *ABCG2-482T* (MCF-7/T8), and "R7" represents MCF-7 cell with introduced *ABCG2* of the present application (MCF-7/R7).
Fig.6 shows the relative resistance to each of anti-cancer drugs of MCF-7 cell to which *ABCG2* of the present application or *ABCG2-482T* gene was introduced. In the drawing, "T8" represents MCF-7 cell with introduced *ABCG2-482T* (MCF-7/T8), and "R7" represents MCF-7 cell with introduced *ABCG2* of the present application (MCF-7/R7).
Fig.7 shows the amount of the accumulation of each of the drugs in MCF-7 cell to which *ABCG2* of the present application or *ABCG2-482T* gene was introduced. In the drawing, "vector" represents MCF-7 cell with introduced the vector only, "T8" represents MCF-7 cell with introduced *ABCG2-482T* (MCF-7/T8), and "R7" represents MCF-7 cell with introduced *ABCG2* of the present application (MCF-7/R7).

### Preferred Embodiments

The present invention relates to a novel drug resistance-associated gene and a product thereof. The nucleotide sequence of cDNA encoding a protein, "ABCG2 of the present invention" (human ABCG2), which has been isolated by the present inventors and which confers resistance to cancer chemotherapeutic drugs on a mammalian cell, is shown in SEQ ID NO:1, and the amino acid sequence of the protein, "ABCG2 of the present invention", coded by the cDNA is shown in SEQ ID NO:2. The cDNA sequence of ABCG2 of the present invention has been discovered based on the screening of a gene that is highly expressed in a cell resistant to Compound-A which is an indolocarbazole compound, and has a two nucleotide difference compared with the sequence disclosed as *BCRP* among the ABCG2 sub-family which belongs to ABC transporter superfamily. Thus, the cDNA of ABCG2 of the present invention encodes a novel protein having an amino acid sequence different from the BCRP in one amino acid residue.

However, in the course of solving the problems of the present invention, it was confirmed that the degree of resistance to various drugs and the amount of intracellular drug accumulation of cell lines transfected with *ABCG2* gene of the present invention were different from those of cell lines transfected with a gene disclosed as *BCRP*. In a cell line over-expressing ABCG2 or transfected with *ABCG2* gene of the present invention, the resistance selective to indolocarbazole compounds was observed. Namely, it has been proved that the *ABCG2* of the present invention is a novel drug resistance-associated gene encoding a protein having a different character from that of BCRP by substituting one amino acid residue thereof.

In the present invention, the term "mammalian cells" means tissues or cells that constitute animal body belonging to mammal, or external cell cultures of such cells.

In the present invention, the term "cancer chemotherapeutic drug" means a drug used for the purpose of treating cancers, and includes a synthetic compound, natural compound derived from plants or microorganisms, and semi-synthetic compound synthesized from a natural compound. In a preferred embodiment, the "cancer chemotherapeutic drug" refers to a compound represented by the following general formula (I): 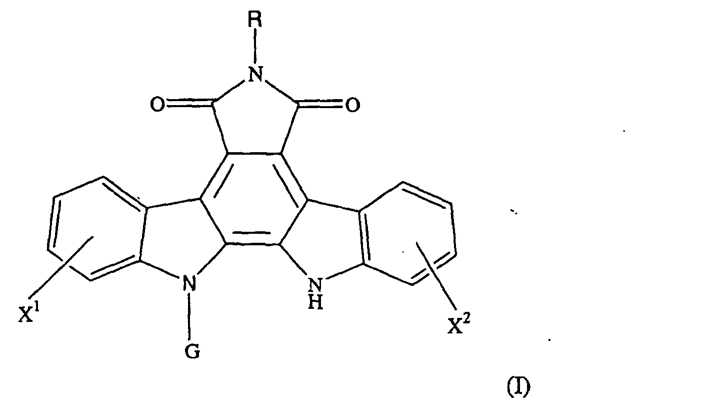 wherein X¹ and X² each independently represent a hydrogen atom, halogen atom or hydroxyl group; R represents a hydrogen atom, amino, formylamino, or lower alkylamino which may be substituted with any one selected from the group consisting of one to three hydroxyl group(s), a pyridyl group optionally having substituent(s), and thienyl group optionally having substituent(s); and G represents a pentose group, hexose group or derivative thereof which may be substituted with an amino group (hereinafter, generically called as indolocarbazole compound). In a more preferred embodiment of the present invention, it refers to a compound represented by the aforementioned general formula (I), wherein X¹ and X² each independently represent a halogen atom or hydroxyl group; R represents a hydrogen atom, formylamino, or lower alkylamino which may be substituted with any one selected from the group consisting of one to three hydroxyl groups, a pyridyl group optionally having substituent(s), and thienyl group optionally having substituent(s); and G represents a hexose group which may be substituted with an amino group. The production method and the like of the aforementioned indolocarbazole compounds have been disclosed in the prior patent applications and registered patents (European patent publication 0528030A1, U. S. Patent Nos. 5591842, 5668271, and 5804564, WO95/30682, WO96/04293, WO98/07433, JP Patent Kokai Publication No. JP-A-10-245390).

The term "resistance to cancer chemotherapeutic drugs" means that a mammalian cell shows the resistance to the therapeutic effect of cancer chemotherapeutic drugs. It means resistance of a cancer cell based on the enhanced efflux activity of cancer chemotherapeutic drugs out of the cancer cell, the mutations of the target enzyme in the cancer cell, the reduction of activity of drug activating systems for prodrugs *in vivo*, the enhancement of damage repair system of the cancer cell, and the like. In a preferred embodiment, the resistance is caused by the decrease of the intracellular drug concentration due to the enhanced efflux of cancer chemotherapeutic drugs out of a cancer cell.

The term "to confer the resistance to cancer chemotherapeutic drugs on a mammalian cell" means giving the enhanced activity to excrete cancer chemotherapeutic drugs out of a cell to a mammalian cell. In a preferred embodiment, it means that this enhanced activity to excrete cancer chemotherapeutic drugs out of a cell is imparted by an amplification of the ABCG2 gene of the present invention belonging to the ABCG2 sub-family included in ABC transporter superfamily (ATP binding cassette superfamily: Higgin, C. F. (1992) *Annu. Rev. Cell. Biol.* 8., 67-113), an increase of the transcript of the ABCG2 gene of the present invention, or an overexpression of the translated product of the *ABCG2* gene of the present invention. In addition, the term "to confer the resistance to cancer chemotherapeutic drugs on a mammalian cell" means that a mammalian cell obtains the resistance to cancer chemotherapeutic drugs, due to the decrease of intracellular concentration of the cancer chemotherapeutic drugs in consequence of active efflux of the cancer chemotherapeutic drug incorporated into the mammalian cell by the function of *ABCG2* gene product of the present invention.

The "ABCG2 protein of the present invention" includes a naturally occurring protein and also a recombinant protein prepared by recombinant DNA technologies. A naturally occurring protein can be prepared, for example, by subjecting tissue extract such as placenta which are supposed to express the "ABCG2" protein of the present invention to affinity chromatography using after-mentioned "anti-ABCG2 antibody", whereas a recombinant protein can be prepared by cultivating a cell which is transformed with a DNA encoding human "ABCG2" protein of the present invention as is described below. For the skilled person in the art, it is capable of performing a modification such as an amino acid replacement in the human ABCG2 protein of the present invention (SEQ ID NO: 2) using known methods to prepare a modified protein having an equivalent function of the protein of the present invention, that is, a binding activity to or a transporting activity of indolocarbazole compounds. The mutation of an amino acid sequence may occur naturally. A protein in which one or several amino acids in a natural protein are replaced, deleted or added and which have an equivalent function to the natural protein, is also included in the present invention. As a method for modifying amino acid residues which are known to the skilled person in the art, there are enumerated, for example, Kunkel method (Kunkel, T. A. *et al., Methods Enzymol*. 154, 367-382 (1987)), Double primer method (Zoller, M. J. and Smith, M., *Methods Enzymol.* 154, 329-350 (1987)), cassette mutagenesis method (Wells, et al., Gene 34, 315-23 (1985)), mega-primer method (Shaker, G. and Sommer, S. S., *Biotechniques* 8, 404-407 (1990)). The number and position of mutated amino acids of a protein are not limited so far as the function of the protein is maintained, however, the number of mutated amino acid(s) in a functionally equivalent protein is generally within 10 % of total amino acids, preferably within 10 amino acids, and more preferably within 3 amino acids (for example, 1 amino acid) and the amino acid at position 482 of the protein of the present invention is arginine.

The present invention also includes a partial peptide of the aforementioned human ABCG2 protein of the present invention. For example, the partial peptide includes but is not limited to a partial peptide having a ATP binding region of ABCG2 protein of the present invention (amino acid residues 61 to 270), a partial peptide having an arginine at the 482nd residue in the amino acid sequence of the protein of the present invention, a partial peptide having a binding activity with indolocarbazole compounds, a partial peptide having an activity to excrete indolocarbazole compounds out of cells when it is expressed on the surface of a cell. These partial peptides can be used, as is the case with the ABCG2 protein of the present invention, for example, for preparing an antibody, screening a candidate compound of the drug as described below, or screening a compound that enhances the therapeutic efficacy of indolocarbazole compounds. Moreover, a partial peptide which has a binding activity to indolocarbazole compounds, without any drug excreting activity out of a cell, may be a competitive inhibitor of the ABCG2 protein of the present invention. Such partial peptides of the present invention may have at least 15 amino acid residues, preferably 20 amino acid residues or more in chain length. The partial peptides of the present invention can be prepared, for example, by a genetic engineering technique, the peptide synthesis method known in the art, or the digestion of the protein of the present invention with appropriate peptidases.

The present invention also relates to a polynucleotide encoding the protein of the present invention or partial peptides thereof. As used herein, the term "polynucleotide" generally refers to both polyribonucleotide and polydeoxyribonucleotide, which can be either a unmodified RNA or DNA, and either a modified RNA or DNA. There is enumerated, for example, DNA, cDNA, genomic DNA, mRNA, unprocessed RNA and a fragment thereof, without particular restriction of its length.

The term "a polynucleotide encoding a protein or partial peptide thereof" refers to a polynucleotide having any nucleotide sequence based on the degeneracy of genetic codes, provided that each sequence can encode the ABCG2 protein of the present invention or partial peptide thereof. As such polynucleotides, for example, there are provided a DNA encoding the ABCG2 protein of the present invention or partial peptide thereof and an RNA such as mRNA. These may be double stranded or single stranded. In the case of double stranded, it may be double stranded DNA, double stranded RNA or DNA:RNA hybrid. The DNA encoding the ABCG2 protein of the present invention or partial peptide thereof includes a genomic DNA, genomic DNA library, cDNA derived from the aforementioned cells and tissues, and synthetic DNA.

The cDNA encoding the ABCG2 protein of the present invention can be screened, for example, by labeling the cDNA represented in SEQ ID NO: 1 or a fragment thereof; complementary RNA thereto, or synthetic oligonucleotides including a portion of the cDNA sequence, with ³²P or the like, and hybridizing them with a cDNA library derived from a tissue expressing the ABCG2 protein of the present invention (e.g. placentae). Alternatively, the cDNA can be cloned by synthesizing oligonucleotides corresponding to the cDNA sequence, and amplifying template cDNA derived from the appropriate tissue (e.g. placentae) by polymerase chain reaction. The genomic DNA can be screened by, for example, labeling the cDNA represented in SEQ ID NO: 1 or a fragment thereof, a complementary RNA thereto, or a synthetic oligonucleotide including a portion of the cDNA sequence, with ³²P or the like, and then hybridizing to the genomic DNA libraly. Alternatively, the genomic DNA can be cloned by synthesizing oligonucleotides corresponding to the cDNA sequence and amplifying the genomic DNA as the template by polymerase chain reaction. On the other hand, a synthetic DNA can be prepared by, for example, chemically synthesizing oligonucleotides having a partial sequence of the cDNA represented, in SEQ ID NO: 1, annealing them to form double strand, and ligating them with DNA ligase (Khorana, H. G. *et al., J. Biol. Chem.* 251, 565-570 (1976); Goeddel D. V. *et al., Proc Natl. Acad. Sci. U.S.A.* 76, 106-10 (1979)).

The polynucleotide of the present invention can be also used for detecting a mammalian cell having resistance to the compounds represented in the aforementioned general formula (I). The detection method includes extraction of mRNA from a mammalian cell as a sample by a general method and measurement of the amount of ABCG2 mRNA by any method such as northern hybridization using the polynucleotide of the present invention as a probe, or performing RT-PCR using a primer set that can hybridize to the polynucleotide sequence of the present invention of the extracted RNA. In this case, the overexpression of the mRNA may be an indication to detect a mammalian cell having resistance to the compound represented in the aforementioned general formula (I).

In order to detect the mRNA or genomic DNA characteristic to the ABCG2 protein of the present invention, a polymorphism corresponding to the codon 482 encoding arginine residue of the ABCG2 protein can be detected. As a method of detecting such a polymorphism, for example, the mRNA extracted from a selected cancer cell may be hybridized with DNA chips and the like on which polynucleotides consisting of 15 to 100 contiguous nucleotides of the sequences of SEQ ID NO:1 and comprising a nucleotide at position 1489 (G, a polymorphic site) of the DNA represented in SEQ ID NO: 1, or complement thereof, is immobilized to detect the nucleotide sequence of the aforementioned polymorphic site.

The DNA of the present invention is useful for the production of the recombinant protein. Namely, it is possible to prepare the human ABCG2 protein of the present invention as a recombinant protein by constructing an appropriate expression vector with the inserted DNA encoding the ABCG2 protein of the present invention (e.g. the cDNA represented in SEQ ID NO: 1), cultivating the transformant obtained by introducing the vector into an appropriate cell, and purifying the expressed protein.

In another embodiment, the present invention relates to a recombinant vector comprising a polynucleotide of the present invention. The term "recombinant vector" refers to a DNA in which an exogenous DNA is incorporated and which can replicate in the host cell. These are prepared by modifying plasmids, phages or viruses which are all self-replicative. As a plasmid and the like which is used for preparing a recombinant vector of the present invention, there is no particular limitation if it can stably hold the inserted polynucleotide. For example, in a case where the host is *Escherichia coli*, a plasmid vector such as pET-3 (Rosenberg, A. H., *et al., Gene* 56, 125-35 (1987)), pGEX-1 (Smith, D. B. and Johnson, K. S., *Gene* 67, 31-40 (1988)) is used. In a case where the host is a fission yeast, *Schizosaccharomyces pombe,* a plasmid vector such as pESP-1 (Lu, Q. et *al., Gene* 200, 135-144 (1997)) is used. In a case where the host is an insect cell, a baculovirus vector pBacPAK8/9 (Clontech) and the like are used. On the other hand, in a case where the host is a mammalian cell such as a Chinese hamster ovary (CHO) cell and a human HeLa cells, vectors such as pMSG (Amersham Pharmacia Biotech.), pcDNA (Invitrogen Corporation) and the like are used.

In still another embodiment, the present invention relates to a transformant comprising the recombinant vector of the present invention. The term "transformant" refers to a cell in which the exogenous DNA is incorporated by a recombinant vector. The host cell in which the recombinant vector of the present invention is introduced may be a prokaryotic cell or a eucaryotic cell, and includes any host cell which can be used for the object of the present invention, such as bacterial cell, yeast cell, insect cell, and mammalian cell. The specific methods for introducing a recombinant vector into the host cell, and obtaining the transformants are as follows. Transformation of *E. coli*. is performed by the method of Hanahan (Hanahan, D., *J. Mol. Biol*. 166, 557-580 (1983)), the electroporation (Dower, W. J. *et al., Nucl. Acids Res*. 16, 6127-6145 (1988)) and the like. Transformation of yeast is performed by, for example, spheroplast method (Beach, D. and Nurse, P., *Nature* 290, 140 (1981)), lithium acetate method (Okazaki, K. *et al*., *Nucleic Acids Res.* 18, 6485-6489 (1990)). Transformation of an insect cell is performed by, for example, a method described in *Bio*/*Technology*, 6, 47-55 (1980). Transfection of a recombinant DNA into a mammalian cell is performed by calcium phosphate method (Graham, F. L. and van der Eb, A. J., *Virology* 52, 456-467 (1973)), DEAE-dextran method (Sussman, D.J. and Milman, G., Mol. Cell. Biol. 4, 1641-1643 (1984)), lipofection method (Felgner, P.L. *et al., Proc. Natl. Acad. Sci. USA* 84, 7413-7417 (1987)), electroporation (Neumann, E. *et al., EMBO J*. 1, 841-845 (1982)) and the like.

In a further embodiment, the present invention relates to a method for producing the protein of the present invention or partial peptides thereof by using the transformant of the present invention. A recombinant protein expressed by the transformant can be isolated from the transformant or culture supernatant thereof according to a standard method of the art. The standard method in this context includes ammonium sulfate precipitation, column chromatography (for example, ion exchange, gel filtration, and affinity chromatography) and electrophoresis. In addition, the recombinat protein can be purified by, for example, synthesizing it as a fusion protein attached at N-terminus with histidine residue tag sequence or glutathione S-transferase (GST) and the like, and binding it to metal chelating resin or GST affinity resin (Smith, M.C. *et al., J. Biol. Chem.* 263, 7211-7215 (1988)), respectively. When pESP-1 is used as a vector, for example, the protein of interest is synthesized as a fusion protein with glutathione 5-transferase (GST), therefore, the recombinant protein can be purified by -binding it to GST affinity resin. The fusion protein may be cleaved with thrombin, blood coagulation factor Xa, and such, to liberate the protein of interest from the fusion protein.

In a still further embodiment of the present invention, an antibody which binds to the human ABCG2 protein of the present invention is provided. The antibody that binds to the protein of the present invention can be prepared according to the method known to the skilled person in the art (e.g. refer to "New experimental course of biochemistry 1, Protein I, 389-406, Tokyo Kagaku Dojin"). The preparation of polyclonal antibody is performed, for example, as follows. To an immunocompetent animal such as rabbit, guinea pig, mouse and chicken is injected the ABCG2 protein of the present invention or partial peptide thereof. The injection may be accompanied by the adjuvant (FIA or FCA) which promotes the antibody production. Administration in usually carried out every several weeks. The titer of antibodies can be elevated by multiple immunizations. After the final immunization, antiserum is obtained by collecting blood from the immunized animal. The polyclonal antibody can be prepared from this antiserum by, for example, fractionation with ammonium sulfate precipitation or anion exchange chromatography, or by affinity purification using Protein A or immobilized antigen. On the other hand, a monoclonal antibody is prepared, for example, as follows. The ABCG2 protein of the present invention or partial peptide thereof is immunized to an immunocompetent animal as described above, and after the final immunization, spleen or lymph node is harvested from the immunized animal. A hybridoma cell is prepared by the cell fusion of the antibody producing cell which is contained in this spleen or lymph node and a myeloma cell using polyethylene glycol and the like. By screening and cultivating the target hybridoma cell, the monoclonal antibody can be prepared from the culture supernatant. The monoclonal antibody can be purified by, for example, fractionation with ammonium sulfate precipitation or anion exchange chromatography, or by affinity purification using Protein A or immobilized antigen. The antibody thus prepared is used for affinity purification of the ABCG2 protein of the present invention And also, it may be used for detecting the amount of the expression of the ABCG2 protein of the present invention as well. The detection of the expression amount of human ABCG2 protein in a mammalian cell by the antibody makes it possible to determine the resistance of the mammalian cell to the compound represented in the aforementioned general formula (I), wherein the resistance is indicated by the overexpression of the protein. In addition, the detection of the human ABCG2 protein in a cancer cell or cancer patient by the antibody can be used for diagnosing the disease or drug resistance caused by the overexpression of the protein. This antibody can be further used for the antibody therapy against those diseases or drug resistance.

It is preferable to use humanized antibodies or human antibodies for the antibody therapy. A humanized antibody, for example, a mouse-human chimeric antibody can be prepared by, for example, isolating the gene encoding the antibody against the ABCG2 protein of the present invention from antibody-producing mouse cells, replacing the constant region on the H chain of the antibody with the human IgE, and introducing into a mouse myeloma cell J558L, (Neuberger, M. S. *et al., Nature* 314, 268-270 (1985)). Alternatively, human antibody can be prepared by immunizing mice whose immune systems have been replaced with that of humans with the ABCG2 protein of the present invention.

In an embodiment, the present invention relates to an antisense nucleotide suppresses the expression of the protein of the present invention. The "antisense nucleotide(s)" of the present invention includes a sequence which specifically hybridizes to any region of mRNA encoding the ABCG2 protein of the present invention or partial peptide thereof, or any region of 5' or 3' untranslated region of the mRNA, and is capable of preventing the translation of the mRNA. The antisense nucleotide(s) can contain a sequence which is capable of specifically hybridizing with any region of the cDNA sequence encoding the ABCG2 protein of the present invention and partial peptide thereof. Specifically, the antisense nucleotide(s) can be designed based on the nucleotide sequence represented in SEQ ID NO:1. It can consist of a complementary sequence of any sequence of coding region or untranslated region of the represented sequence. The antisense nucleotide of the present invention may be RNA, DNA or modified nucleic acid (RNA, DNA), and may contain modified sugars, bases or linkages. The specific examples of the modified nucleic acid includes but is not limited to a sulfurized derivative, thiophosphate derivative and the like.

The inhibitory activity of the antisense nucleotide can be evaluated by using the transformant of the present invention, *in vivo* or *in vitro* gene expression system of an ABCG2 protein, or, *in vivo* or *in vitro* translation system of an ABCG2 protein. The antisense nucleotide can be placed in the cell through any number of ways known per se. For example, the antisense nucleotide can be delivered in the specialized systems such as liposome or microsphere, or may have attached moieties. Such attached moieties include polycationic substance as polylysine which act as charge neutralizers of the phosphate backbone, or hydrophobic substance as lipids (for example, phospholipids, cholesterols) that enhance interaction with cell membrane and increase uptake of nucleic acid. It is applicable to gene therapy as well as cultured cell. As well as by the introduction of the oligonucleotide itself from the outside as described above, the antisense nucleotide(s) can be also performed through the mediation of a vector and the like which is capable of expressing the antisense nucleotide *in vivo.* In the Japanese patent Kohyo publication JP-A-11-511965, it has been shown that an antisense oligonucleotide which suppresses the expression of human genes *MDR1* and *MRP* encoding multi-drug resistance associated proteins enhanced the sensitivity of a multi-drug resistant cell to the drugs. According to the method described in this prior art, the sequence of the antisense nucleotides of the present invention can be designed by selecting an appropriate target region. The antisense nucleotides of the present invention can be used for enhancing the sensitivity to an indolocarbazole compound by administering it to a drug-resistant cancer cell or cancer patient in which the ABCG2 protein of the present invention is overexpressed.

In another embodiment of the present invention, there is provided a method for predicting the resistance of a patient to cancer chemotherapeutic drugs, wherein the resistance is indicated by the expression of the protein of the present invention. The term "expression" in the phrase "indicated by the expression" includes both meanings of the process transcribed from DNA to mRNA and the process further translated to a polypeptide or protein.

The term "indicated by the expression of the protein" means that the resistance to cancer chemotherapeutic drugs is indicated by comparing the amount of mRNA of the protein or the amount of the protein (polypeptide) itself in the sample with those of surrounding normal tissues or culture cells of known drug resistance to assess the amount of the expressed protein. In a specific embodiment of the present invention, the comparison of the amount of mRNA is performed by extracting RNA by routine methods from a mammalian cell, cancer cell of a patient, tissue preparation, tissue sample or the like, and assaying by northern hybridization, as is shown in Example 4, with DNA fragment comprising a partial sequence of the polynucleotide encoding the ABCG2 protein of the present invention as a probe. Alternatively, the amount of mRNA of the protein is determined by performing RT-PCR according to the literatures (Noonan, K. E., *et al., Proc. Natl. Acad. Sci. USA* (1990) 87, 7160-4 and Futscher, B. W., *et al., Anal Biochem* (1993) 213, 414-21) by using the extracted RNA and a primer set which can hybridize to the polynucleotide sequence encoding the ABCG2 protein of the present invention. The methods which measure the amount of mRNA, however, are not limited to these methods.

In the case of measuring and comparing the amount of the protein (polypeptide) itself, the method includes but is not limited to the comparison to the control by the immunohistochemical staining of a mammalian cell, cancer cell of a patient or tissue section, according to the method of the literature (Beck, W. T., *et al., Cancer Res.* (1996) 56, 3010-20) with the aforementioned "antibody", or by the western blotting using a standard method with respect to the protein extracts of the sample.

When the protein is observed to be overexpressed in the sample compared to controls by any method described above or another, the cell or the patient derived from the sample is decided to be resistant to cancer chemotherapeutic drugs.

The present invention also includes a method for screening a compound which inhibits the function of the ABCG2 protein of the present invention. This is a method which searches any one of substances as follows. (i) a substance which inhibits the binding of the ABCG2 protein of the present invention to a substrate compound thereof. (ii) a substance which inhibits the activation of ATPase activity of the ABCG2 protein of the present invention by binding with its substrate compound. (iii) a substance which inhibits the membrane transport of a substrate compound by the ABCG2 protein of the present invention. (iv) a substance which enhances the cytotoxicity of a substrate compound of the ABCG2 protein of the present invention.

The screening method of the present invention which searches the substance (i) comprises the steps: (a) contacting the ABCG2 protein of the present invention or partial peptide thereof with its substrate compound in the presence of a candidate compound, and measuring the binding activity between the protein or partial peptide thereof and the substrate compound, and (b) comparing the binding activity detected in the step (a) with that in the absence of the candidate compound and selecting a compound which suppresses the binding activity between the ABCG2 protein of the present invention or partial peptide thereof and the substrate compound. A candidate compound includes but is not limited to a protein, peptide, non-peptide compound, artificially synthesized compound, extract of tissues or cells, serum. The ABCG2 protein of the present invention or partial peptide thereof used for screening does not limited to its purified state but can be various forms, for example, in a form of bound to an affinity column, in a form of a membrane vesicle of a desired cell (including a transformant genetically engineered to express the protein) in which the protein or partial peptide thereof is expressed in the membrane (which can be prepared according to the method of Leier I. *et al., Journal of Biological Chemistry* 269 (45): 27807-10, 1994), or in a form of a reconstituted vesicle of the purified protein or partial peptide on a liposome (which can be prepared according to the method of Anbudkar, S. V. *et al., Proc. Natl. Acad. Sci. USA* (1992) 89: 8472-8476). The substrate compounds used for the screening are not limited to certain substances, but are preferably indolocarbazole compounds, for example, Compound-A, which are used by labeling as necessary. The label includes but is not limited to, for example, a radiolabel, fluorescence label, photoaffinitylabel. The binding activity of the ABCG2 protein of the present invention to the substrate compound can be detected through the labeling of the compound bound to the ABCG2 protein of the present invention (for example, by measuring radioactivity or fluorescence intensity). In case of a photoaffinitylabel, the binding activity can be also detected according to the method of the literature (Cornwell MM. *et al., Proc. Natl. Acad. Sci. USA*. 83:3847-50, 1986) by isolating a covalently bonded complex between the protein or partial peptide thereof and a photoaffinitylabeled compound using SDS polyacrylamide gel and the like, and then measuring the radioactivity of the photoaffinitylabeled compound by autoradiography. As a result of the detection, it is determined that the candidate compound has the inhibitory activity of the binding between the ABCG2 protein of the present invention and the substrate compound, when the binding activity in the presence of the candidate compound is lower than that in the absence of the candidate compound (control experiment).

The screening method of the present invention which searches the substance (ii) comprises the steps: (a) measuring ATPase activity when the ABCG2 protein of the present invention or partial peptide thereof is contacted with a substrate compound in the presence of a candidate compound, and (b) comparing the ATPase activity detected in the step (a) with that in the absence of the candidate compound, and selecting a compound which suppresses the ATPase activity of the ABCG2 protein of the present invention by binding of the substrate compound. The candidate compound includes but is not limited to a protein, peptide, non-peptide compound, artificially synthesized compound, extract of tissues or cells, serum. The ABCG2 protein of the present invention or partial peptide thereof which is used for the screening method can be, for example, in a form of a membrane vesicle of a desired cell (including a transformant genetically engineered to express the protein), in which the protein or partial peptide thereof is expressed in the membrane or in a form of reconstituted form of the purified protein or partial peptide on a liposome. The substrate compounds used for the screening is not limited to certain compounds but are preferably indolocarbazole compounds, for example, Compound-A. The ATPase activity can be measured by a standard method, for example, a colorimetry of amounts of inorganic phosphate produced by hydrolysis of ATP (refer to Adam B. Shapiro *et al., Journal of Biological Chemistry*, 269:3745-3754, 1994). It is decided that the candidate compound has the inhibitory activity of the activation of ATPase activity of the ABCG2 protein of the present invention by the substrate compound, when the ATPase activity in the presence of the candidate compound is lower than that in the absence of the candidate compound (control experiment).

The screening method of the present invention by searching the substance (iii) is classified broadly into three methods.

The first method uses a membrane vesicle of a desired cell (including a transformant genetically engineered to express the protein) in which the ABCG2 protein of the present invention or partial peptide thereof is expressed in the membrane, or through a reconstituted vesicle of the purified protein or partial peptide on a liposome, and comprises the steps: (a) contacting the vesicle with a substrate compound of the protein in the presence of a candidate compound, and then, measuring the amount of the substrate compound which is transported into the vesicle and (b) comparing the amount of the substrate compound transported into the vesicle and measured in the step (a) with that in the absence of the candidate compound and selecting a compound which inhibits the membrane transporting activity of the substrate compound by the ABCG2 protein of the present invention or partial peptide thereof. Specifically, the method can be performed by, for example, a method according to the description in *J. Biol. Chem*. 269, 27807-10 (1994). The substrate compounds used for the screening are not limited to certain compounds but are preferably indolocarbazole compounds, for example, Compound-A, which are used by labeling as necessary. The label includes but is not limited to, for example, a radiolabel, fluorescence label, photoaffinitylabel. The amount of the substrate compound transported into the vesicle is detected by these labels (for example, by measuring radioactivity or fluorescence intensity). As a result of the detection, it is decided that the candidate compound has the inhibitory activity of the membrane transporting activity of the substrate compound by the ABCG2 protein of the present invention or partial peptide thereof, when the amount of the intravesical substrate compound in the presence of the candidate compound is lower than that in the absence of the candidate compound (control experiment).

The second method uses a desired cell (including a transformant genetically engineered to express the protein) in which the ABCG2 protein of the present invention or partial peptide thereof is expressed in (through) the. ··· membrane, and comprises the steps: (a) measuring the amount of a substrate compound after contacting the cell with the substrate compound of the protein in the presence of a candidate compound and (b) comparing the amount of the substrate compound accumulated in the cell and measued in the step (a) with that in the absence of the candidate compound and selecting a compound which inhibits the membrane transporting (excreting) activity of the substrate compound out of the cell by the ABCG2 protein of the present invention or partial peptide thereof.

The third method uses a desired cell (including a transformant genetically engineered to express the protein) in which the ABCG2 protein of the present invention or partial peptide thereof is expressed in(through) the membrane, and comprises the steps: (a) contacting the cell with the substrate compound of the protein to accumulate the substrate compound in the cell, and (b) measuring an amount of the residual substrate compound in the cell after cultivation of certain period of the cell obtained in the step (a) in the presence of a candidate compound, and (c) comparing the amount of the substrate compound accumulated in the cell and detected in the step (b) with that in the absence of the candidate compound, and then selecting a compound which decrease the membrane transporting (excreting) activity of the substrate compound out of the cell by the ABCG2 protein of the present invention or partial peptide thereof. The second and third methods can be performed by a method according to the description in Bruin, M. *et al., Cancer. Let.* 146, 117-26 (1999). In each of the second and third method, the used substrate compounds are not limited to certain compounds, but includes a fluorescent substance which can penetrate into a cell and can be transported by the ABCG2 protein of the present invention, or indolocarbazole compounds, for example, Compound-A, which are used by labeled as necessary. As a label, for example, a radiolabel, fluorescence label, photoaffinitylabel and the like are enumerated. The amount of the substrate compound accumulated or remained in a cell is detected by these labels (for example, by measuring radioactivity in case of a radiolabel, or fluorescence intensity by flow cytometry, fluorescence microscope, fluorophotometer and the like in case bf a fluorescence label). As a result of the detection, it is decided that the candidate compound has an inhibitory activity of the membrane transporting activity of the substrate compound by the ABCG2 protein of the present invention or partial peptide thereof, when the amount of the intracellular accumulation or residue of the substrate compound in the presence of the candidate compound is higher(more) than that in the absence of the candidate compound (control experiment). In any of the first, second and third screening methods, the candidate compound includes but is not limited to a protein, peptide, non-peptide compound, artificially synthesized compound, extract of tissues or cells, serum.

The screening method of the present invention by searching the substance (iv) uses a desired cell (including a transformant genetically engineered to express the protein) in which the ABCG2 protein of the present invention or partial peptide thereof is expressed in(through) the membrane, and comprises the steps: (a) cultivating the cell with a substrate compound of the protein for a constant time in the presence of a candidate compound and measuring the number of viable cells, and (b) comparing the viable cell number measured in the step (a) with that in the absence of the candidate compound, and then selecting a compound which enhances the cytotoxicity of the substrate compound. The candidate compound includes but is not limited to a protein, peptide, non-peptide compound, artificially synthesized compound, extract of tissues or cells, serum and the like. The substrate compounds used for the screening are not limited to certain compounds but are preferably indolocarbazole compounds, for example, Compound-A, which have a cytotoxic effect. It is well known to those of the skilled person in the art that the measurement of the number of viable cells can be replaced by the measurement of the amount of the. protein or that of the activity of mitochondrial reducing enzyme.

Inhibitors to be obtained by the screening method of the present invention as described above can confer suppressed resistance to drugs on a cell and patient that are resistant to the drugs because of efflux of the drugs out of the cell caused by the ABCG2 protein of the present invention. Namely, the inhibitors are useful as drug-sensitivity enhancing agents. Pharmaceutical compositions comprising these compounds may be useful for treatment of diseases associated with membrane transport systems mediated by the ABCG2 protein of the present invention.

Inhibitors obtained by the screening method of the present invention are compounds to inhibit the activity of the ABCG2 protein of the present invention. To be specific, the inhibitor binds with high affinity to the ABCG2 protein and inhibits the molecule from transporting the substrates thereof to the outside competitively or non-competitively. The inhibitor includes a peptide, protein, non-peptide compound, synthesized compound, fermentation product. These compounds may be novel compounds or known ones. These compounds can be administered to a cell expressing the ABCG2 protein together with an anticancer agent which is a substrate for the ABCG2 protein, to enhance the effect of the anticancer agent. This method is particularly useful for treatment for a cancer that aquired anticancer drug resistance through expressing the ABCG2 protein, and thus the inhibitory compound can be a pharmaceutical agent effective in overcoming the drug-resistance.

### Examples

The present invention is explained in more detail by reference to the following examples and reference examples, however, these examples do not restrict the scope of the present invention.

### [Example 1] Establishment of cell lines resistant to Compound-A

To establish Compound-A-resistant cell lines from different cell lines, a total of three cell lines: the mouse fibroblast cell line LY, human lung cancer cell line PC-13, and human colon carcinoma cell line HCT116 were cultivated for a long time in the presence of Compound-A as follows.

### (Example 1-1) Establishment of Compound-A-resistant mouse fibroblast cell line LY/NR1:

LY cells were cultivated in the presence of 0.1 µM Compound-A for 2 weeks, then in the presence of 0.3 µM for 3 weeks. A colony that grew under these conditions was isolated using a cloning ring (Asahi Techno Glass Corporation) and designated as LY/NR1.

### (Example 1-2) Establishment of Compound-A-resistant mouse fibroblast cell line LY/NR2:

LY cells were cultivated in the presence of 0.1 µM Compound-A for 2 weeks, and then in the presence of 0.3 µM for 5 weeks. An emerging colony was isolated as described above and designated as LY/NR2.

### (Example 1-3) Establishment of Compound-A-resistant human cell line PC-13/NR13:

A Compound-A-resistant PC-13X13 cell line isolated after culture of PC-13 cells in the presence of 1.1 µM Compound-A for 5 weeks was cultured in the presence of 20 µM Compound-A for 4 weeks. An emerging colony was isolated and designated as PC-13/NR13.

### (Example 1-4) Establishment of Compound-A-resistant human cell line HCT116/NR1:

A Compound-A-resistant HCT116X13 cell line isolated after culture of the HCT116 cells in the presence of 1.1 µM Compound-A for 5 weeks was cultured in the presence of 20 µM Compound-A for 4 weeks. An emerging colony was isolated and designated as HCT116/NR1.

### (Example 1-5) Selection of a Compound-A-resistant spontaneous cell line:

A single clone was obtained by limiting dilution method from endocervical carcinoma cells, HeLa cells, which were spontaneous resistant to Compound-A, and designated as HeLa#7 cell line. The existing HeLaS3 cell line which is derived from the same original HeLa cells, was used as a Compound-A-sensitive cell for comparative experiments to the HeLa#7.

### [Example 2] Measurement of drug sensitivity of the Compound-A-resistant cell lines to various anticancer drugs:

The sensitivities of the established Compound-A-resistant cell lines to Compound-A, Compound-B, and other anti-cancer drugs were measured using Sulforhodamine B dye-staining (SRB) method as follows.

Test cells in the logarithmic growth phase were dispensed into wells of 96-well plate at 1 X 10³ cells/well. After subculture for 24 hours in a CO₂ incubator, a serial diluted agent was added to each well. After further incubation for 72 hours, the cells were fixed with trichloroacetic acid (TCA) and the proteins in the cells were stained by 0.4% sulforhodamine B solution. The samples were eluted by 10 mM Tris-HCI for 1 hour, and each well was measured at 560 nm with a 450 nm reference, by a SPECTRAmax 250 plate reader (Molecular Devices Corp.). In this specification, IC₅₀ was defined as an agent concentration to inhibit the growth of a cell line to 50%, and Relative Resistance was determined by dividing an IC₅₀ value of an agent to a resistant cell line by an IC₅₀ value of the agent to its parental cell line.

The measurement revealed that all of the Compound-A-resistant human cell lines displayed more than 100-fold resistance to both Compound-A and Compound-B as compared with their parental cell lines (Table 1). The mouse LY/NR1 cell line showed moderate resistance to Compound-A and Compound-B, the Relative Resistance of which were 12 and 17, respectively. The mouse LY/NR2 cell line displayed high resistance to Compound-A and Compound-B, the Relative Resistance of which were 64 and 210, respectively (Table 2).

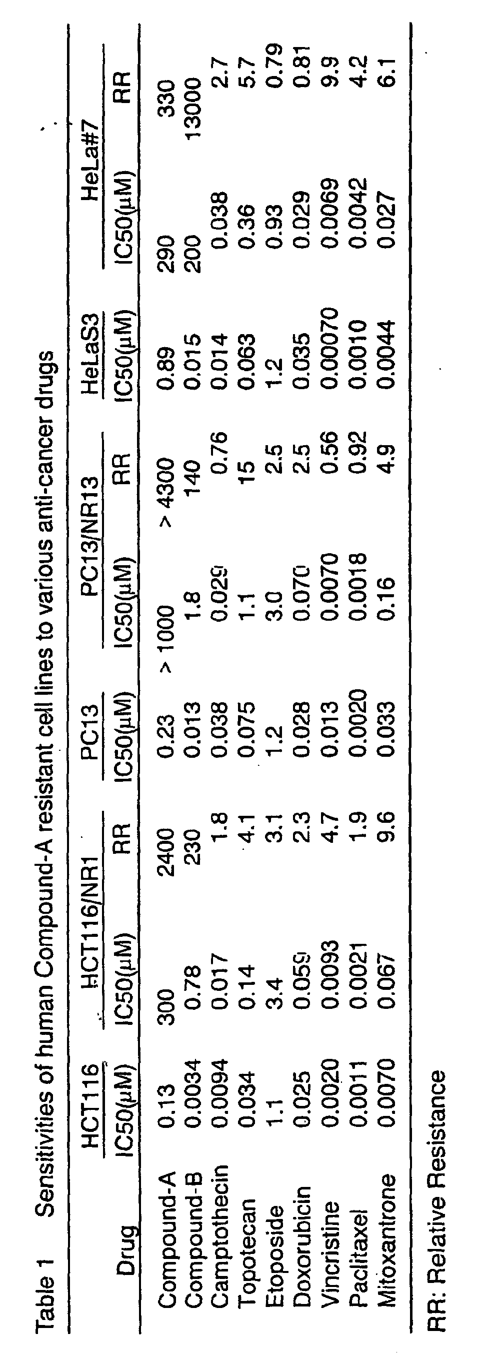

**Table 2**

| Sensitivities of mouse Compound-A resistant cell lines to various anti-cancer drugs | | | | | |
|---|---|---|---|---|---|
| | LY | LY/NR1 | | LY/NR2 | |
| Drug | IC50(µM) | IC50(µM) | RR | IC50(µM) | RR |
| Compound-A | 0.12 | 1.4 | 12 | 7.7 | 64 |
| Compound-B | 0.0017 | 0.029 | 17 | 0.36 | 210 |
| Camptothecin | 0.046 | 0.058 | 1.3 | 0.38 | 8.2 |
| Topotecan | 0.069 | 0.25 | 1.8 | 2.9 | 20 |
| Etoposide | 0.28 | 0.52 | 2.1 | 2.0 | 4.7 |
| Doxorubicin | 0.043 | 0.067 | 1.6 | 0.19 | 4.5 |
| Vincristine | 0.015 | 0.011 | 0.77 | 0.025 | 1.7 |
| Paclitaxel | 0.041 | 0.057 | 1.4 | 0.081 | 2.0 |
| Mitoxantrone | 0.0033 | 0.0071 | 1.5 | 0.057 | 17 |
| RR: Relative Resistance | | | | | |

### [Example 3]

### (Example 3-1) Preparation of RNA from cell lines LY, LY/NR1, and LY/NR2:

RNA from each of the cell lines LY, LY/NR1, and LY/NR2 was prepared by the following method. Approximately 1 x 10⁷ of cells were detached from the culture dish by trypsinization and collected by centrifugation. The cells were then homogenized using a QlAshredder (QIAGEN Inc.) and total RNA was isolated using an RNeasy total RNA isolation kit (QIAGEN Inc.).

### (Example 3-2) Preparation of cRNA:

A complementary DNA was synthesized from 32 µg of each total RNA fraction supplemented with a primer, T7-(dT)₂₄ (SEQ ID NO: 3: GGCCAGTGAATTGTAATACGACTCACTATAGGGAGGCGG-(dT)₂₄), using Superscript II Reverse Transcriptase (BRL) and a supplied buffer. Then the double stranded DNA was synthesized by adding *E.coli* DNA Ligase (BRL), *E. coli* DNA Polymerase I (BRL), *E. coli* RNaseH (BRL) and a supplied buffer in the reaction mixture and incubating at 16 °C for 2 hours, followed by the incubation with T4 DNA Polymerase (BRL). After treating the reaction mixtures with phenol:chloroform (1:1) and precipitating the treated mixtures with ethanol, the precipitates were solubilized in 12 µl of distilled water. A cRNA was synthesized by adding T7 RNA Polymerase (ENZO) and biotinylated UTP and CTP to 2.5 µl of. the ds cDNA fraction, and then fragmented by acid treatment.

### (Example 3-3) Hybridization, washing and staining on DNA microarray:

Fifteen micrograms of the fragments of these cRNAs were solubilized in a buffer containing 100 mM MES, 1 M [Na+], 20 mM EDTA, and 0.01% Tween 20, and subjected to hybridization on DNA microarrays at 45 °C for 16 hours. In this example, GeneChips: Mu11KsubA, subB, Mu19KsubA, subB, and subC (Affymetrix Inc.) were used as the microarrays. DNA microarray wash procedure was carried out according to the Affymetrix Fluidics Station protocol. Thereafter, each of the DNA microarrays was stained by antibody fluorescence amplification method as follows. First the DNA microarrays were stained by adding Streptavidin-modified Phycoerythrin (Molecular Probes, Inc.), Then reacted with goat IgG (SIGMA) and biotinylated goat anti-Streptavidin antibody (Vector Laboratories, Ltd.), and re-stained with Streptavidin-modified Phycoerythrin.

### (Example 3-4) DNA microarray Data Analysis:

After the hybridization, washing and staining, the DNA microarrays were subjected to measurement of the fluorescence intensities using GeneChip scanner (Hewlett-Packard). The scanning was repeated two times and the average of the results was stored as image data. Quantification of expression levels and comparative analysis thereof were performed by analyzing the image data by GeneChip software (Affymetrix Inc.). The expression levels of the genes were compared between LY and LY/NR2 and between LY and LY/NR1 by Comparison Analysis of the above software.

As a result, it was an *ABCG2* gene of ABC transporter family members that, among approximately 30,000 kinds of genes analyzed concerning their expression levels, showed the most elevated expression selective to LY/NR2 cell line in comparison with that in LY. The ABCG2 gene showed 31-fold increase in expression compared with the parental cell line LY (Table 3). The result suggests that the resistance to Compound-A of each cell line correlated with the increase of this gene expression.

**Table 3**

| Analysis of genes with selectively elevated expressions in mouse Compound-A resistant cell lines by DNA chips | | |
|---|---|---|
| | Fold increase in resistant cells^{a} (fold) | |
| Gene Description | LY/NR2 | LY/NR1 |
| ABCG2 | 31.2 | 6.0 |
| at070537 Full Length w/o function | 15.5 | 13.4 |
| ATP Synthetase A chain | 6.2 | NC^{b} |
| Interferon Activatable protein | 5.2 | NC |
| Interferon Activatable protein | 4.7 | 2.3 |
| 3-beta hydroxylstroid dehydrogease | 4.6 | NC |
| Interferon Activatable Protein | 4.5 | 4.7 |
| EST | 4.5 | NC |
| Y13275, Meta-associ tetraspan molecule | 4.3 | 3.8 |
| 19kD Glycoprotein Autoantigen | 4.2 | 3.2 |
| Lipocortin, x07486 | 4.1 | NC |
| Ig heavy chain precursor | 4.0 | NC |
| Y-box transcription factor | 4.0 | -1.6 |

| | | |
|---|---|---|
| a) The genes that showed more than 4-fold elevated expression in LY/NR2 compared with that in LY cell are listed in decreasing order of their elevated expression. Among these genes, genes displaying the change of expression in LY/NR1 list their value of the elevated expression compared with that in LY cell in the column LY/NR1. b) Genes showing no change of expression compared with that in their parental cells are represented as NC. | | |

### [Example 4] Northern Blot Analysis

To confirm the selective expression of *ABCG2* in the resistant cell lines LY/NR1 and Ly/NR2 detected by DNA microarray analysis, Northern blot analysis was performed using cDNA fragment of mouse *ABCG2* as a probe, and the same was performed using cDNA fragment of human *ABCG2* of the present invention as a probe to detect the expression of human ABCG2 in Compound-A-resistant human cell line.

### (Example 4-1) Preparation of Blots

A total RNA was extracted from each of the cell lines LY, LY/NR1, LY/NR2, HeLaS3, HeLa#7, HCT116, HCT116/NR1, PC-13, and PC-13/NR13 by guanidine isothiocyanate method using ISOGEN reagent (Nippon Gene, Japan). From the obtained total RNA, poly (A)⁺ RNA was purified using FastTrak® 2.0 Kit (Invitrogen Corp.). For detection of each gene's expression, 0.8 µg of the poly (A)⁺ RNA obtained from each cell line was electrophoresed on a formaldehyde denatured agarose gel and transferred to Hybond N+ membrane (Amersham Pharmacia) using 20 X SSC buffer for overnight. After transfer, the membranes were air-dried and the nucleic acids on the membranes were UV-fixed by STRATALINKER (Stratagene, Inc.).

### (Example 4-2) Preparation of probes and Hybridization

For detection of mouse *ABCG2*, the following procedure was employed to obtain a mouse ABCG2 cDNA fragment used as a probe. PCR amplification of the cDNA was performed using 2 µl of the LY/NR2 cDNA prepared in Example 3 as a template, with an upstream DNA primer (SEQ ID NO:4: CTCATTTAAAAACTTGCTCGGGAACC) and a downstream DNA primer (SEQ ID NO:5: CAAGAGGCCAGAAAAGAGCATCATAA) synthesized based on the mouse ABCG2 DNA sequence. The 50 µl of PCR reaction mixture was composed of 200 nM each of the synthetic DNA primers, 0.1 mM dNTPs, 0.5 µl of Ex Taq DNA polymerase (TaKaRa, Japan) and 5 µl of a buffer attached to the enzyme. The amplification was performed for 29 cycles using the thermal cycler (PerkinElmer, Inc.). Each cycle consisted of 30 sec at 95ºC, 1 min at 60ºC, and 2 min at 70ºC. Then, after diluting the amplified product, a further PCR amplification was performed using an upstream DNA primer (SEQ ID NO: 6: TACTGGGGCTTATTATTGGTG) and a downstream DNA primer (SEQ ID NO: 7: AAAAGCGATTGTCATGAGAAGTGT) which were annealed to sequences of the amplified product. In the reaction, an amplification was performed for 35 cycles. Each cycle consisted of 30 sec at 95ºC, 30 sec at 62ºC, and 2 min at 72ºC. The amplified products were confirmed by 1% agarose gel electrophoresis and ethidium bromide staining. This mouse *ABCG2* cDNA fragment was purified by QlAquick PCR Purification Kit (QIAGEN) and radiolabeled with [α-³²P] dCTP using Multiprime Labelling Kit (Amersham Pharmacia) to prepare a probe for hybridization.

On the other hand, for detection of human *ABCG2* gene, PCR amplification was performed using a plasmid having a full length human *ABCG2* cDNA of the present invention shown in Example 5, as a template, with an upstream DNA primer (SEQ ID NO:8: CAAAAAGCTTAAGACCGAGCTCTATTA AGC) and a downstream DNA primer (SEQ ID NO:9: ATCCTCTAGACCAGG TTTCATGATCCCATTG). The amplified cDNA fragment was radiolabeled by the same method that was performed on the mouse cDNA, to prepare a probe for hybridization. Each of these cDNA probes and 1 mg of salmon sperm DNA were added to the membranes which were prehybridized in QuikHyb hybridization solution (Stratagene) at 65ºC for 30 min, then hybridization was performed at 65ºC for 1 hour. Thereafter, the membranes were washed with 2 X SSC containing 0.1% SDS at room temperature for 30 min, then with 0.1 X SSC containing 0.1% SDS at room temperature for 15 min, and finally with 0.1 X SSC containing 0.1% SDS at 65ºC for 10 min. The radioactivities of the washed membranes were measured by BAS5000 Image Analyzer (Fuji Photo Film Co.) and processed for imaging. In order to clarify that approximately equal amounts of sample RNAs were loaded, each blot was further hybridized with a GAPDH (glyceraldehyde-3-phosphate dehydrogenase) probe: the membranes hybridized with the *ABCG2* probe were immersed for 1 min in boiled solutions containing 0.5% SDS and left until they reached room temperature to remove the probes on the membranes. Then the membranes were hybridized with a human GAPDH DNA fragment as a probe, washed and measured by the same conditions that were described in hybridization with *ABCG2* probe.

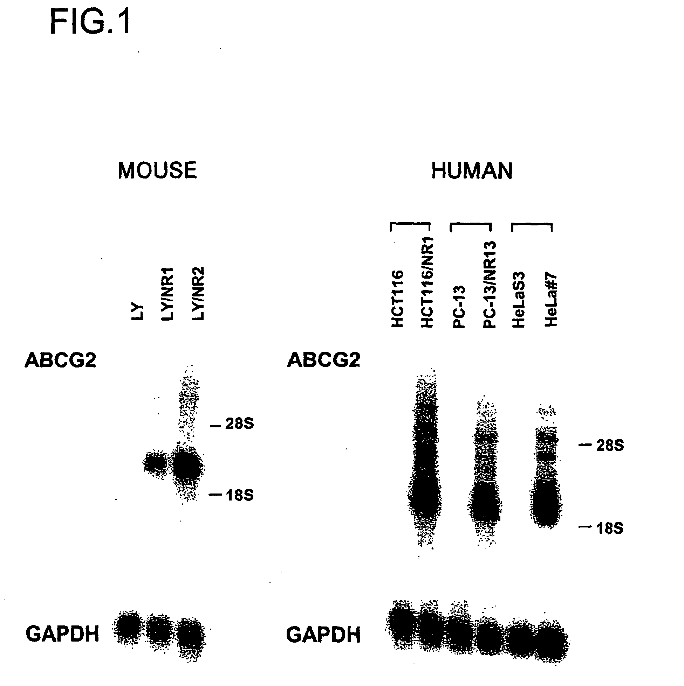

As a result, the mouse cell lines LY, LY/NR1 and LY/NR2 showed an elevated expression of the *ABCG2* gene correlated with an increase of resistance, as was demonstrated in the DNA microarray analysis. It was also found that the human *ABCG2* gene was selectively overexpressed in all Compound-A-resistant human cell lines such as HCT116/NR1, PC-13/NR13 and HeLa#7, which strongly suggests that the gene of the present invention is involved in Compound-A and Compound-B resistance (Fig. 1).

### [Example 5] Isolation of full length human ABCG2 cDNA from HeLa #7 Cell

### (Example 5-1) Preparation of synthetic DNA primer sequences based on the known ABCG2 sequence

A synthetic DNA primer sequence for PCR, flanking both initiation and termination codons, was prepared based on the sequence reported as ABCP mRNA (GenBank: AF103796). 5' primer (SEQ ID NO:8: CAAAAAGCTTAAGACCGAGCTCTATTAAGC) 3' primer (SEQ ID NO:10: GAATTAAGGGGAAATTTAAGAAT) In 5' primer, eight bases, which are required for digestion by the restriction enzyme Hind III, were added to 5' end of the sequence derived from ABCP.

### (Example 5-2) Preparation of total RNA fraction from HeLa#7 cell and Synthesis of cDNA

Total RNA was prepared from the cultured human cell line HeLa#7 by guanidine isothiocyanate method using ISOGEN reagent (Nippon Gene, Japan). Then the complementary DNA was synthesized from 2.5 µg of the total RNA fraction by Superscript II Reverse Transcriptase (BRL) with 18 bases of oligo dT as a primer and a supplied buffer.

### (Example 5-3) Amplification of human ABCG2 gene by PCR using the cDNA derived from HeLa#7 cell and Determination of its nucleotide sequence

Amplification by PCR using the synthetic primers was performed using 1µl (125 ng) of the cDNA prepared from the HeLa#7 cells in Example 5-2 as a template. The 50 µl of reaction mixture was composed of 300 nM each of the synthetic DNA primers (SEQ ID NO:8 and No.10), 0.2 mM dNTPs, 2 µl of LA Taq DNA polymerase (TaKaRa, Japan) and 5 µl of a buffer attached to the enzyme. The amplification was performed 30 cycles using the thermal cycler (PerkinElmer, Inc.). Each cycle consisted of 30 sec at 94ºC, 1 min at 55ºC, and 4 min at 72ºC. The amplified product was confirmed by 1% agarose gel electrophoresis and ethidium bromide staining.

### (Example 5-4) Subcloning of the PCR product into a plasmid vector and Determination of nucleotide sequence of the inserted cDNA region

The reaction product obtained by PCR in Example 5-3 was separated using 1% agarose gel and the region containing its band was cut out from the gel by a knife, followed by recovery of the DNA using QlAquick Gel Extraction Kit (QIAGEN). After adding A to the recovered DNA at its 3' end by rTaq DNA polymerase, the DNA was subcloned into a plasmid vector pcDNA3.1/V5-His-TOPO according to a method of Eukaryotic TOPO TA cloning Kit (Invitrogen Corp.). *E. coli* TOP10 competent cells (Invitrogen Corp.) were transformed by the plasmid, then clones having the cDNA insert were selected in LB agar medium containing ampicillin. Emerging colonies were isolated by sterilized picks. Then PCR using a primer set spanning a multicloning site of the plasmid vectors was performed for each clone, and clones having inserts with predicted size were selected. The clones with the PCR product of interest inserted was cultured for overnight in LB medium containing ampicillin, a plasmid DNA was prepared using QIAprep 8 Turbo miniprep kit (QIAGEN). The reaction for determining the nucleotide sequence was performed using DyeDeoxy Terminator Cycle Sequencing Kit (ABI) and the nucleotide sequence was determined by means of a fluorescence automatic sequencer.

As a result, it was found out that the sequence of *ABCG2* full length cDNA isolated from HeLa#7 cell was nearly identical to those of *ABCG2* cDNAs reported previously except for several essential differences.

The nucleotide sequence of human *ABCG2* of the present invention was shown in SEQ ID NO:1 and the *E.coli* strain, designated as *"E.coli* HELabcg2, into which the full length cDNA was cloned was deposited as follows:
(i) Name and Address of the Depositary Institution
   NAME: International Patent Organism Depositary, National institute of Advanced Industrial Science and Technology (formerly National Institute of Bioscience and Human-Technology, Agency of Industrial Science and Technology, MITI)
   ADDRESS: Tsukuba Central 6, 1-1-1 Higashi, Tsukuba, Ibaraki, Japan (postcode: 305-8566)
(ii) Date of Deposit (the original date): September 25, 2000
(iii) Accession number of Domestic Deposit: No. 18053 (FERM P-18053)
(iv) Date of Transfer to Budapest Treaty Deposit: September 5, 2001
(v) Accession number of Budapest Treaty Deposit: FERM BP-7726

There are two different *ABCG2* genes previously reported about their full length sequences: *ABCP* and *BCRP*. These two genes have different parts in their sequences. Where are concerned only the differences of sequences involving in replacement of amino acid, the sequences differ in the regions coding amino acids at positions 24, 166, 208, and 482. *ABCP* and *BCRP* code valine and alanine as amino acid at position 24, glutamic acid and glutamine at position 166, serine and phenylalanine at position 208, and arginine and threonine at position 482, respectively.

Among the above four regions, the sequence of the gene the inventors obtained is identical to that of *BCRP* concerning the regions coding amino acids at positions 24, 166, and 208, i.e. the present invention's gene codes alanine, glutamine, and phenylalanine as amino acids at positions 24, 166, and 208, respectively, while only one region coding amino acid at position 482, i.e. arginine, of the present gene is identical to that of *ABCP*. Namely, the sequence of the gene the inventors obtained is identical to that reported as *BCRP* except for one amino acid at position 482, i.e. the gene is a single amino acid substituent of *BCRP*.

### [Example 6] Amplification of human ABCG2 cDNA by PCR using a cDNA derived from normal human tissue and Determination of the nucleotide sequence thereof

Concerning region different from the sequences of *ABCG2* or *BCRP,* the nuclleotide sequence of *ABCG2* cDNA derived from normal human tissue was determined using direct sequencing analysis.

PCR amplification was performed using synthetic DNA primers (SEQ ID NO:8: CAAAAAGCTTAAGACCGAGCTCTATTAAGC and SEQ ID NO:11: AGAGATCGATGCCCTGCTTTACCA), and 2.5 µl (∼0.5 ng) of cDNA derived from placenta and kidney contained in Human MTC™ Panel I (Clontec) as a template. The 50 µl of PCR reaction mixture was composed of 200 nM each of the synthetic DNA primers (SEQ ID NO:8 and No.11), 0.4 mM dNTPs, 0.5 µl of LA Taq DNA polymerase and 5 µl of a buffer attached to the enzyme. The amplification reaction was performed for 35 cycles using the thermal cycler (PerkinElmer, Inc.). Each cycle consisted of 30 sec at 94ºC, 1 min at 55ºC, and 3 min at 72ºC. The amplified product was confirmed by 1% agarose gel electrophoresis and ethidium bromide staining. The PCR product was purified by QlAquick PCR Purification Kit (QIAGEN), then subjected to direct sequencing. The reaction for determining the nucleotide sequence, as performed in Example 5-4, was performed using DyeDeoxy Terminator Cycle Sequencing Kit (ABI) and the sequence was determined by means of a fluorescence automatic sequencer.

As a result, it was found out that in the *ABCG2* cDNA derived from human placenta and kidney, the regions coding amino acids at positions 24, 166, 208, and 482 code alanine, glutamine, phenylalanine, and arginine, respectively, which suggests that the same sequence as the inventors obtained from HeLa#7 cells is also expressed in normal human tissue.

### [Example 7] Preparation of human ABCG2 expressing cells

As shown in Example 5, the human *ABCG2* full length cDNA from HeLa#7 cell was inserted into a plasmid for expression in mammalian cells: pcDNA3.1-/V5-His-TOPO (Invitrogen Corp.). PC-13 cells were transfected with the *ABCG2* expression plasmids using Effectene Transfection Reagent (QIAGEN). The experiment was performed according to the attached procedural manual. As a control experiment, other PC-13 cells were transfected with vector alone without the *ABCG2* sequence. The two experiments were conducted simultaneously. Two days after the transfection, the original media were replaced with selection media containing 0.2 mg/ml of Geneticin (GIBCO BRL). By culture for a long time under this condition, stable transfectant clones with the introduced plasmids were selected. Two weeks from the transfection, emerging colonies were picked and expression of ABCG2 in the cells was examined by Northern Blot analysis. Several clones with high level of expression of *ABCG2* such as clones PC-13/ABCG2-2, PC13/ABCG2-3 were selected and subjected to subsequent analysis. Northern Blot analysis was performed as described in Example 4: 8 µg of total RNA prepared from each clone using RNeasy mini kit (QIAGEN) was separated on a formaldehyde denatured agarose gel, followed by analysis using human *ABCG2* cDNA fragment of the present invention as a probe.

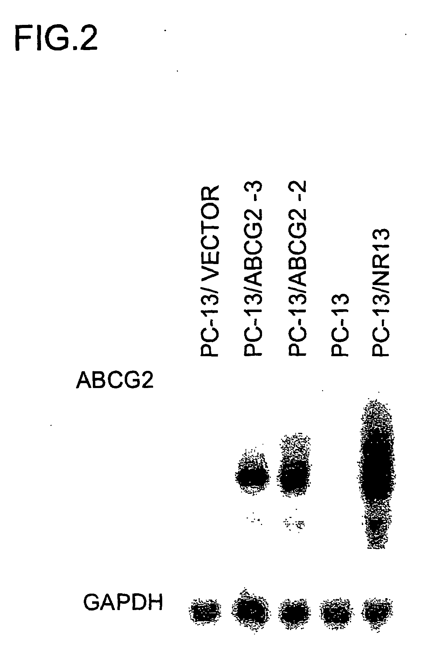

As a result, it was found that the clones PC-13/ABCG2-2 and PC-13/ABCG2-3 highly expressed a transcript of the present invention's ABCG2. The expression level in the clone PC-13/ABCG2-2 was about 35% of that in the resistant cell line PC-13/NR13 and the expression level in the clone PC-13/ABCG2-3 was about 20% of that in the resistant cell line PC-13/NR13 (Fig.2).

### [Example 8] Measurement of Drug Sensitivity to Various Cancer Chemotherapeutic Drugs of Human ABCG2 Expressing Cells

The drug sensitivity of the stable transfectant clone, PC-13 cell with transfected human ABCG2 expressing plasmid, was measured by SRB method described in Example 2.

As a result, the PC-13 cell with introduced vector alone did not show resistance to the two compounds (Compound-A and Compound-B). On the other hand, the clone PC-13/ABCG2-2 with the highest expression of introduced ABCG2 showed 22- and 17-fold resistance to Compound-A and Compound-B, respectively, and the clone PC-13/ABCG2-3 with the second highest expression showed 9- and 11.7-fold resistance to Compound-A and Compound-B, respectively, compared to the PC-13 cell with introduced vector alone. These results strongly suggest that the overexpression of ABCG2 confers resistance to these indolocarbazole compounds on the cells. However, these PC-13 cell lines with introduced *ABCG2* did not display remarkable resistance to the other agents, i.e. camptothecin, topotecan, mitoxantrone, and etoposide (table 4). PC-13/ABCG2-2 and PC-13/ABCG2-3 displayed 0.42- and 0.60-fold resistance, respectively, to mitoxantrone compared to the PC-13 with introduced vector alone, which is much different from the report that MCF-7 cells expressing introduced BCRP showed 30-fold resistance to mitoxantrone (Doyle, A. *et al., Proc. Natl. Acad. Sci. U. S. A.* 95, 15665-15670 (1998)).

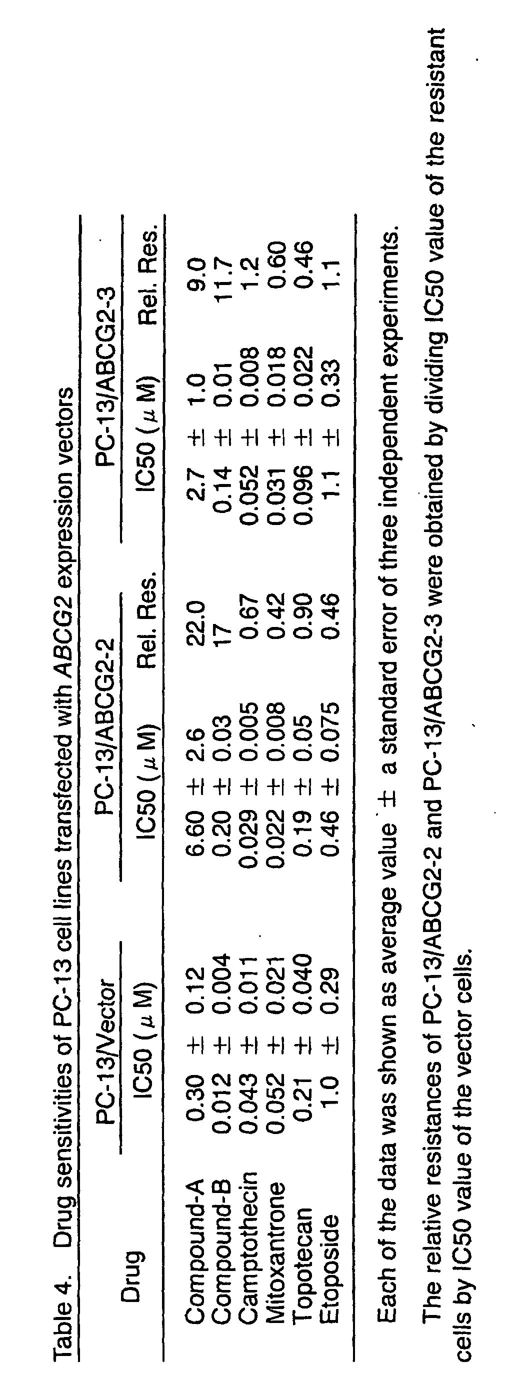

### [Example 9] Analysis of indolocarbazole Compound Accumulation in Cells expressing Human ABCG2 of the Present Invention:

The amount of accumulation of Compound-B in stable transfectant clone, PC-13 cell with transfected *ABCG2* expression plasmid were measured as follows.

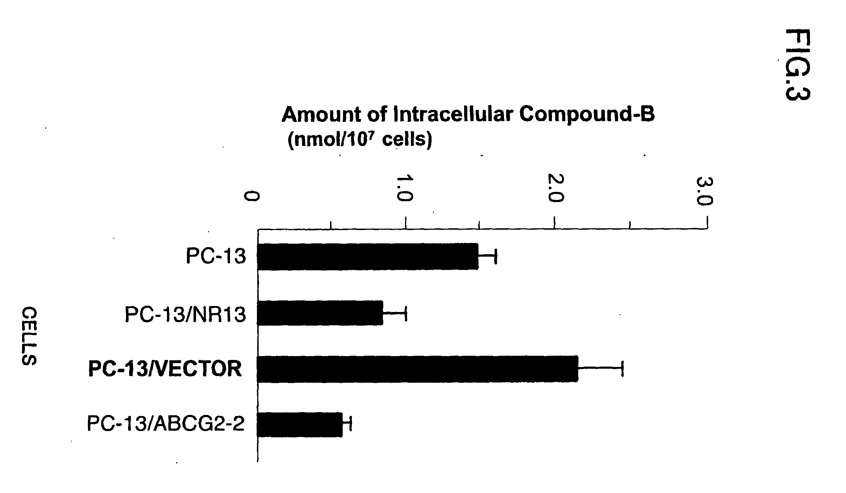

The cells seeded at a density of 1.5 X 10⁶ in a 25-cm² culture flask were incubated for overnight. On the following day, the original medium was replaced with a medium containing 50µM [¹⁴C]-labeled Compound-B, followed by culture at 37ºC for 120 min in a CO₂ incubator to incorporate the labeled compound into the cells. After incubation for 120 min, the cells were rapidly chilled on ice, washed with PBS, detached using 2.5% trypsin. The lysate was centrifuged at 400 X g at 4ºC for 3 min to collect the cells. The cells again suspended in PBS were counted and centrifuged under the same conditions that were described above to recover the cells again. The cells were lysed by Triton X-100 and centrifuged at 2000 X g at 4ºC for 15 min to collect the fractions of the membrane and cytoplasm as supernatant. The remaining pellet was solubilized in 0.2 N NaOH to lyse the nuclei. The obtained supernatant and nuclei samples were supplemented with Clear-sol I (Nacalai Tesque, Inc.) and separately counted in a liquid scintillation counter: TRICARB2300 (Packerd). The values of the supernatant and nuclei samples were added together as Intracellular Accumulation (Fig.3).

As a result of this analysis, the accumulation of Compound-B in PC-13/ABCG2-2 cells with introduced the *ABCG2* expression plasmid is almost the same level as that in the Compound-A resistant cell PC-13/NR13 cell line, while the value was about only one quarter of that in cells with introduced vector alone. The result shows that the cells expressing ABCG2 with the sequence identified by the present inventors displayed an extremely reduced accumulation of Compound-B, which strongly suggests the efflux of indolocarbazole compounds by genes of the present application.

### [Example 10] Preparation of ABCG2-482T sequence by introducing a base substitution

In order to clearly differentiate between the activities of the present invention's *ABCG2* (SEQ ID NO:1) whose codon at position 482 codes arginine and the gene reported above as *BCRP*, reported by Doyle *et al*., whose codon at position 482 codes threonine, a *BCRP*-type full length *ABCG2* cDNA with a single base substitution introduced was prepared, according to the following procedure, from the cDNA sequence of the cloned *ABCG2* gene in Example 5, designated as *ABCG2-482T* (SEQ ID NO: 12). Note that in reference to amino acid sequence, the sequence of *ABCG2-482T* is the same as that of the gene registered as *BCRP,* although in reference to nucleotide sequence, there is another position a single base is changed without substitution of amino acid.

### (Example 10-1) Preparation of a synthesized cDNA primer sequence with a single base substitution introduced in order to change to ABCG2-482T sequence

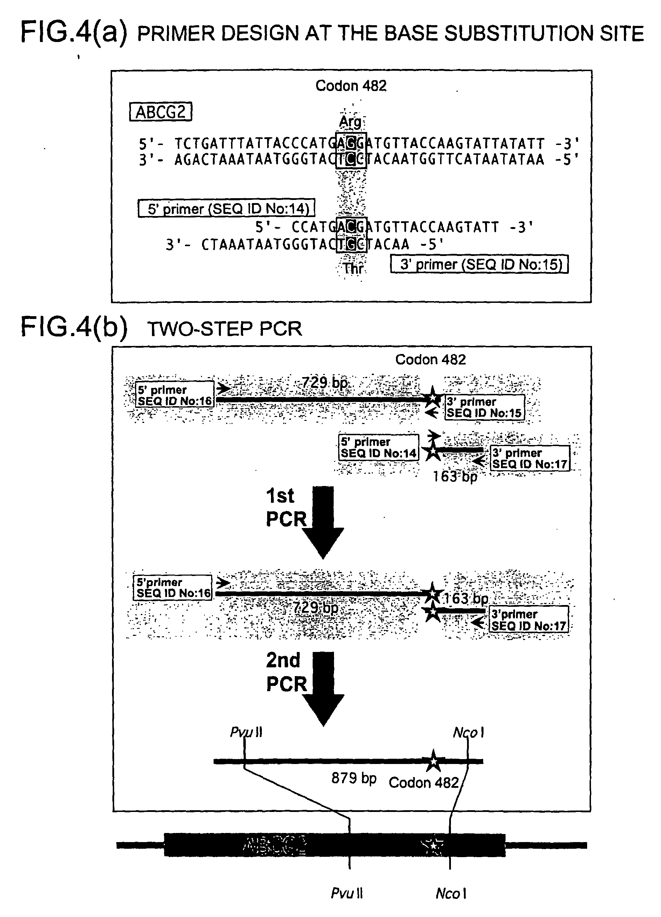

Firstly, based on the cDNA sequence of the present invention's *ABCG2* gene, two DNA primers for PCR were synthesized. Each of the two primers had a sequence in which the nucleotide sequence of the *ABCG2* gene's codon at position 482 was replaced with the corresponding region of the nucleotide sequence reported as *BCRP*, and could anneal the corresponding strand of the *ABCG2* cDNA (see Fig.4-(a)). Note that the lowercase character in the following sequence of each primer represents a single base substitution.

5' primer (SEQ ID NO: 14: CCATGAcGATGTTACCAAGTATT)

3' primer (SEQ ID NO: 15: AACATCgTCATGGGTAATAAATC)

### (Example 10-2) Introduction of a single base substitution by two steps PCR

Next, cDNA fragments of the upstream region and of the downstream region, each of which contained the region where the substitution should be introduced, were separately prepared by PCR using the plasmid encoding the ABCG2 gene cDNA in Example 5 as a template.

The cDNA fragment of the upstream region was amplified by PCR using a 5' primer (SEQ ID NO: 16: CATTCATCAGCCTCGATATTCCA) and a 3' primer (SEQ ID NO: 15: AACATCgTCATGGGTAATAAATC) prepared in Example 10-1. The cDNA fragment of the downstream region was amplified by PCR using a 5' primer prepared in Example 10-1 (SEQ ID NO: 14: CCATGAcGATGTTACCAAGTATT) and a 3' primer (SEQ ID NO: 17: ACCACACTCTGACCTGCTGCTA) [Fig.4-(b), 1st PCR].

So as to link between these two cDNA fragments, the mixture of the two cDNAs was then amplified by PCR using a 5' primer (SEQ ID NO: 16: CATTCATCAGCCTCGATATTCCA) and a 3' primer (SEQ ID NO: 17: ACCACACTCTGACCTGCTGCTA) [Fig.4-(b), 2nd PCR] so that a PCR product including the base substitute region inside was obtained. After PCR, the reaction product was separated using 1% agarose gel and the region containing its band was cut out from the gel by a knife, followed by collection of the DNA using QIAquick Gel Extraction Kit (QIAGEN). After adding A to the collected DNA at its 3' end by rTaq DNA polymerase, the DNA was subcloned into a plasmid vector pCR2.1-TOPO according to a protocol of Eukaryotic TOPO TA cloning Kit (lnvitrogen Corp.). *E. coli* TOP10 competent cells (Invitrogen Corp.) were transformed by the plasmids, then clones having the cDNA insert were selected in LB agar medium containing ampicillin. Emerging colonies were isolated by sterilized picks. PCR using a primer set spanning a multicloning site of a plasmid vectors was then performed for each clone, and clones having an inserts with predicted size were selected. The clones with the PCR product of interest inserted was cultured for overnight in LB medium containing ampicillin, a plasmid DNA was prepared using QlAprep 8 Turbo miniprep kit (QIAGEN). A reaction for determining the nucleotide sequence was performed using DyeDeoxy Terminator Cycle Sequencing Kit (ABI) and the nucleotide sequence was decoded by means of a fluorescence automatic sequencer. As a result, the introduction of a single base substitution was revealed.

### (Example 10-3) Preparation of full length ABCG2-482T cDNA and Introduction thereof into a plasmid capable of expressing in animal cells

The cDNA fragment with a single base substitution introduced prepared in Example 10-2 was digested by two restriction enzymes, *Pvu* II and *Nco* I, which cut the inside of the fragment, and the fragment including the region with the introduced substitution was isolated. By ligating this fragment to a fragment obtained by digesting the *ABCG2* cDNA cloned in Example 5 using *Pvu* II and *Nco* I, a full length *ABCG2* cDNA was obtained whose region including codon 482 was replaced with a fragment in which the substitution was introduced. The full length cDNA was designated as *ABCG2-482T*.

By inserting the *ABCG2-482T* cDNA into a plasmid vector pcDNA3.1/Myc-His, a plasmid expressing in animal cells was prepared. *E. coli* TOP10 competent cells (Invitrogen Corp.) were transformed by the plasmids, then clones having the cDNA insert were selected in LB agar medium containing ampicillin. Emerging colonies were isolated by sterilized picks. PCR using a primer set spanning a multicloning site of the plasmid vectors was then performed for each clone, and clones having insert with predicted size were selected. The clones with the PCR product of interest inserted was cultured for overnight in LB medium containing ampicillin, a plasmid DNA was prepared using QIAprep 8 Turbo miniprep kit (QIAGEN). A reaction for determining the nucleotide sequence was performed using DyeDeoxy Terminator Cycle Sequencing Kit (ABI) and the nucleotide sequence was decoded by means of a fluorescence automatic sequencer, confirming that the plasmid had the sequence of *ABCG2-482T*.

### [Example 11] Preparation of MCF-7 cell which expresses ABCG2 of the present invention or ABCG2-482T

The full length human *ABCG2* cDNA of the present invention prepared in Example 5 was inserted into a plasmid for expression in mammalian cells, followed by transfection of human breast cancer cells, MCF-7 cells, with the *ABCG2* expression plasmid using Effectene Transfection Reagent (QIAGEN). Likewise, human breast cancer cells, MCF-7 cells, were transfected with a plasmid including the *ABCG2-482T* cDNA prepared in Example 10. The experiments were carried out according to an attached procedural manual. As a control experiment, the MCF-7 cells were also transfected with vector alone. The three experiments were conducted simultaneously. Two days from the transfection, the original media were replaced with selective media containing 900 µg/ml of Geneticin (GIBCO BRL). By culture over a prolonged term under this condition, stable transfectant clones with introduced plasmid were selected. Two to three weeks from the transfection, emerging colonies were picked and the expression of *ABCG2* in the cells was examined by Northern Blot analysis. Northern Blot analysis was performed as described in Example 4: 8 µg of total RNA prepared from each clone using RNeasy mini kit (QIAGEN) was separated on a formaldehyde denatured agarose gel, followed by analysis using the human *ABCG2* cDNA fragment of the present invention as a probe.

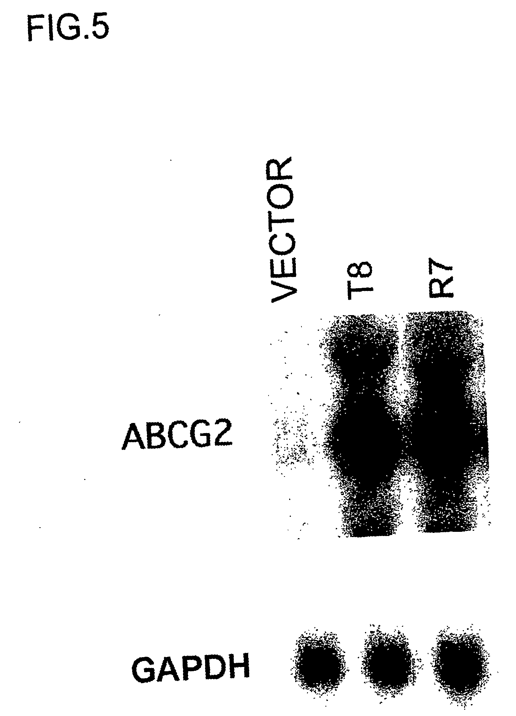

As a result, it was found that MCF-7/R7 among the clones transfected with *ABCG2* of the present invention and MCF-7/T8 among the clones transfected with *ABCG2-482T*, a single amino acid substituent, showed high expression of their respective introduced genes. The two clones were subjected to subsequent analysis. The two clones displayed almost the same amounts of expression of *ABCG2* (Fig.5).

### [Example 12] Measurement of Drug Sensitivity to Various Cancer Chemotherapeutic Drugs of MCF-7 cell which expresses ABCG2 of the present invention or ABCG2-482T

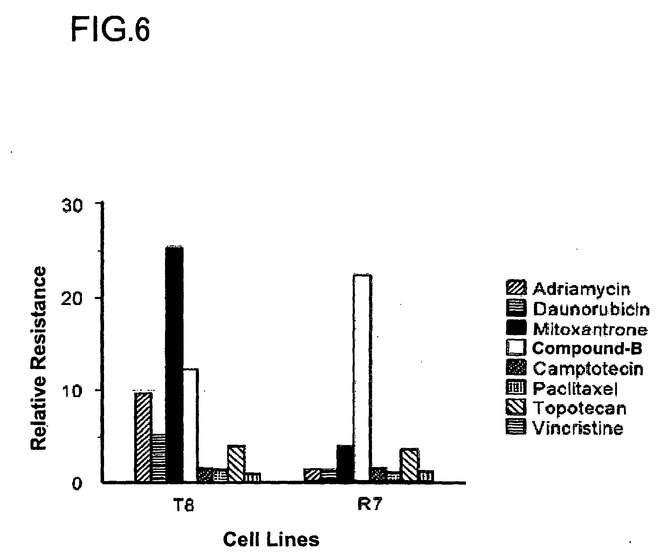

The drug sensitivity of the stable transfectant clones which were established from MCF-7 cells with introduced *ABCG2-* or *ABCG2-482T*-expression plasmid, was measured using SRB method as described in Example 2 and the sensitivity of each transfectant to drugs was shown as a relative resistance to the cells with introduced vector alone (Fig.6).

As a result, MCF-7/T8 with introduced *ABCG2-482T* displayed 12-fold resistance to Compound-B, and also showed resistance to several compounds, i.e. 25-, 9.6-, and 5.1-fold resistance to Mitoxantrone, Adriamycin, and Daunorubicin, respectively. Namely, as was reported previously as BCRP, it was demonstrated that the cell expressing genes whose codon 482 codes threonine has a property as a multidrug resistance factor, i.e. responsible for resistance to various compounds.

On the contrary, MCF-7/R7 with introduced *ABCG2* of the present invention showed high resistance, i.e. 22-fold resistance to Compound-B, but displayed mere 3.8-fold resistance to Mitoxantrone and no resistance to Adriamycin and Daunorubicin. Namely, it was found out that cells expressing the present invention's ABCG2 whose codon 482 codes arginine is selectively resistant to Compound-B.

As was described above, it was demonstrated that ABCG2 of the type reported as BCRP confers resistance to a broad range of chemotherapeutic drugs, while the present invention's ABCG2 different from BCRP-ABCG2 in one amino acid confers resistance selective to indolocarbazole.

### [Example 13] Analysis of indolocarbazole Compound Accumulation in MCF-7 cell which expresses ABCG2 of the present invention or ABCG2-482T

The amount of accumulation of Compound-B in the MCF-7 stable transfectant clones with introduced *ABCG2* of the present invention or *ABCG2-482T* was measured as follows.

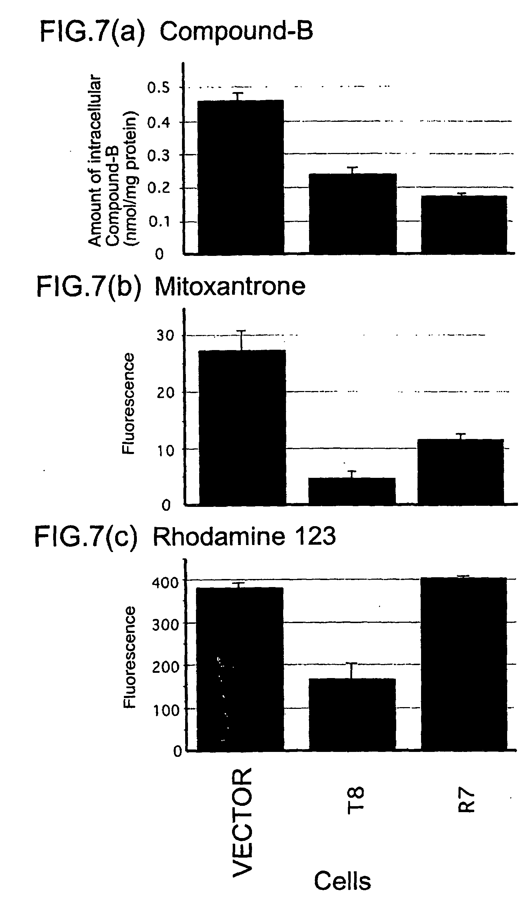

Cells seeded at 8 X 10⁵ cells/well in a 6-well culture plate were incubated for overnight. The next day, the cells in a fresh medium containing 7µM [¹⁴C]-labeled Compound-B were cultured at 37ºC for 180 min in a CO₂ incubator to incorporate the labeled compound into the cells. After 180 min incubation, the cells were rapidly chilled on ice, washed with PBS three times, then the cells were lysed by adding 2N NaOH and incubating at 40ºC for 60 min with shaking. Portions of the obtained lysate samples were subjected to the determination of protein concentration with Bradford method. The remaining parts of the samples were measured in a liquid scintillation counter: TRICARB2500 (Packerd) after addition of Hionic-fluor (Packerd). The result was indicated as Intracellular Accumulation [Fig.7-(a)].

As a result of this analysis, MCF-7/T8 with introduced *ABCG2-482T* and MCF-7/R7 with introduced the present invention's *ABCG2* both showed reduced accumulation of Compound-B, which proved that the. cell with introduced *ABCG2-482T* and that with introduced the present invention's gene both perform active efflux of indolocarbazole compounds.

### [Example 14] Analysis of Mitoxantrone and Rhodamine Accumulation in MCF-7 cell which expresses ABCG2 of the present invention or ABCG2-482T

The amount of accumulation of Mitoxantrone in the MCF-7 stable transfectants with introduced *ABCG2* of the present invention or *ABCG2-482T* was measured as follows.

Cells seeded at 1.0 X 10⁶ cells/well in a 6-well culture plate were incubated for overnight. The next day, the cells in a fresh medium containing 20µM Mitoxantrone were cultured at 37ºC for 90 min in a CO₂ incubator to incorporate the compound into the cells. After a lapse of 90 min, the cells were rapidly chilled on ice, washed once with PBS, detached using 2.5% trypsin. The lysate was centrifuged at 400 X g at 4ºC for 3 min to collect the cells. The collected cells were suspended in Hank's Balanced Salt Solutions containing 1 % BSA and subjected to fluorimetry of the compound using a flow cytometer, Epics Elite (Beckman Coulter, Inc.). The value was indicated as Intracellular Accumulation [Fig.7-(b)].

On the other hand, the amount of intracellular accumulation of Rhodamine in the MCF-7 stable transfectant clones with introduced ABCG2 of the present invention or *ABCG2-482T* was measured as follows.

Cells seeded at 1.0 X 10⁶ cells/well in a 6-well culture plate were incubated for overnight. The next day, the cells in a fresh medium containing 5µM Rhodamine were cultured at 37ºC for 30 min in a CO₂ incubator to incorporate the compound into the cells. After a lapse of 30 min, the cells were rapidly chilled on ice, washed once with PBS, detached using. 2.5% trypsin. The lysate was centrifuged at 400 X g at 4ºC for 3 min to collect the cells. The collected cells were suspended in Hank's Balanced Salt Solutions with 1% BSA and subjected to fluorometry of the compound using a flow cytometer, FACSCalibur (Becton Dickinson and Company). The value was indicated as Intracellular Accumulation [Fig.7-(c)].

As a result of this analysis, it was found out that accumulation of Mitoxantrone in MCF-7/T8 cell was reduced to about 20% of that in the cell with introduced vector alone, suggesting strong efflux of Mitoxantrone in MCF-7/T8 cell, whereas the accumulation in MCF-7/R7 cell was only reduced to about 50% of that in the cell with introduced vector alone, suggesting not so strong efflux in MCF-7/R7 cell. On the contrary, it was found out that accumulation of Rhodamine in MCF-7/T8 cell was reduced to about 50% of that in the cell with introduced vector alone, whereas the accumulation in MCF-7/R7 cell was almost the same level as that in the cell with introduced vector alone, suggesting no efflux of Rhodamine in MCF-7/R7 cell.

As was described above, it was demonstrated that attributed by substitution of a single amino acid, *ABCG2* of the present invention and *ABCG2-482T* are different in their substrate-selectivity from each other.

### Industrial Applicability

The present invention provides a protein that selectively transports indolocarbazole compounds outside the cell and the gene thereof. This enables screening of inhibitors using the transporter protein and the gene coding it and diagnosis on the suitability for administration of an anticancer drug. In addition, its application to treatment for cancer can be expected because inhibitors to be obtained can enhance the sensitivity of cancer cells to anticancer drugs.

## Claims

1. A protein consisting of an amino acid sequence of the following (a) or (b), and conferring the resistance to a cancer chemotherapeutic drug(s) on a mammalian cell:
(a) an amino acid sequence of SEQ ID NO:2,
(b) an amino acid sequence of SEQ ID NO:2, wherein one or several amino acids are replaced, deleted or added.

2. The protein of claim 1, wherein said cancer chemotherapeutic drug(s) is a compound(s) represented by the following general formula (I): 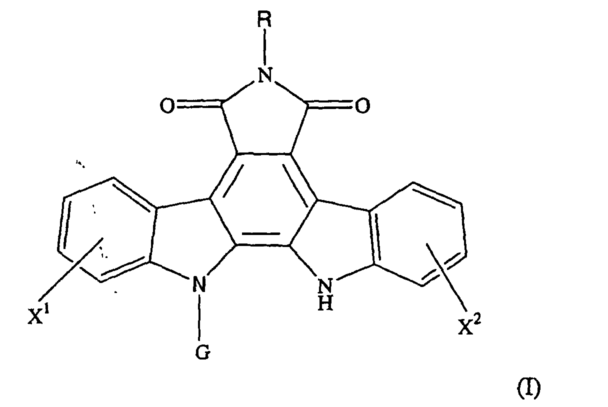 wherein X¹ and X² each independently represent a hydrogen atom, halogen atom or hydroxyl group;
R represents a hydrogen atom, amino, formylamino, or lower alkylamino which may be substituted with any one selected from the group consisting of one to three hydroxyl group(s), a pyridyl group optionally having substituent(s), and thienyl group optionally having substituent(s); and
G represents a pentose group, hexose group or derivative thereof which may be substituted with an amino group.

3. A partial peptide of the protein of claim 1 or 2.

4. A polynucleotide which encodes the protein or the partial peptide of any one of claims 1 to 3, or a complementary polynucleotide thereto.

5. The polynucleotide of claim 4, wherein the DNA sequence of said polynucleotide consists of at least a part of the coding region of SEQ ID NO:1 or the complementary sequence thereto.

6. The polynucleotide of claim 4 or 5, which is used for detecting a mammalian cell having the resistance to a compound represented by the following general formula (I): 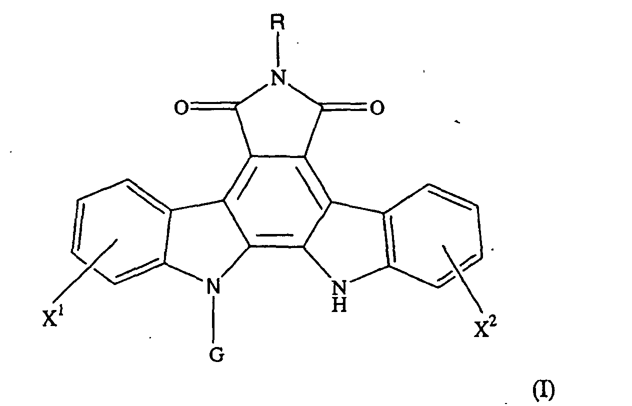 wherein X¹ and X² each independently represent a hydrogen atom, halogen atom or hydroxyl group;
R represents a hydrogen atom, amino, formylamino, or lower alkylamino which may be substituted with any one selected from the group consisting of one to three hydroxyl group(s), a pyridyl group optionally having substituent(s), and thienyl group optionally having substituent(s); and
G represents a pentose group, hexose group or derivative thereof which may be substituted with an amino group..

7. A polynucleotide consisting of a contiguous 15 to 100 DNA sequence of SEQ ID NO:1, said sequence comprising a G at nucleotide number 1489 of the DNA sequence represented in SEQ ID NO:1, or a complementary polynucleotide thereto.

8. A recombinant vector comprising the polynucleotide of claim 4 or 5.

9. A transformant comprising the vector of claim 8.

10. A method for producing the protein or the partial peptide of any one of claims 1 to 3, comprising the steps of:
cultivating the transformant of claim 9, and
recovering the expressed protein or the partial peptide from said transformant or the supernatant thereof.

11. An antibody which specifically binds to the protein or the partial peptide of any one of claims 1 to 3.

12. The antibody of claim 11, which is used for detecting a mammalian cell having the resistance to cancer chemotherapeutic drug represented by the following general formula (I): 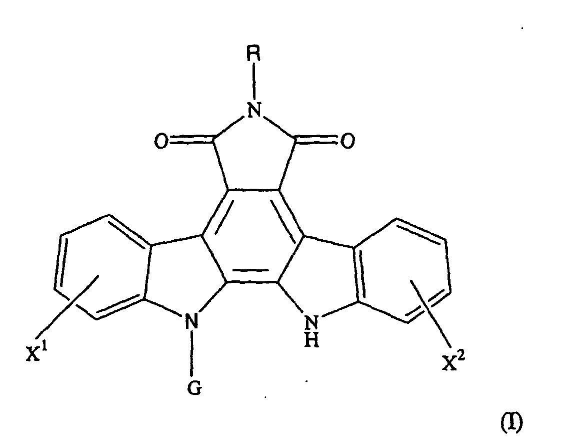 wherein X¹ and X² each independently represent a hydrogen atom, halogen atom or hydroxyl group;
R represents a hydrogen atom, amino, formylamino, or lower alkylamino which may be substituted with any one selected from the group consisting of one to three hydroxyl group(s), a pyridyl group optionally having substituent(s), and thienyl group optionally having substituent(s); and
G represents a pentose group, hexose group or derivative thereof which may be substituted with an amino group..

13. An antisense nucleotide which suppresses the expression of the protein of claim 1 or 2.

14. A method for predicting the resistance to a cancer chemotherapeutic drug of a patient, said resistance being indicated by the expression of the protein of claim 1 or 2.

15. The method of claim 14, wherein said cancer chemotherapeutic drug is a compound represented by the following general formula (I): 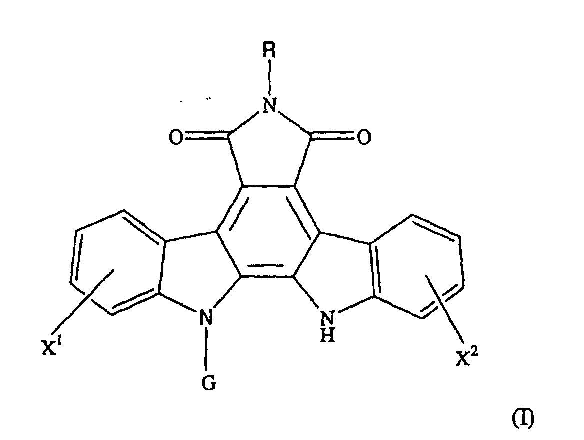 wherein X¹ and X² each independently represent a hydrogen atom, halogen atom or hydroxyl group;
R represents a hydrogen atom, amino, formylamino, or lower alkylamino which may be substituted with any one selected from the group consisting of one to three hydroxyl group(s), a pyridyl group optionally having substituent(s), and thienyl group optionally having substituent(s); and
G represents a pentose group, hexose group or derivative thereof which may be Substituted with an amino group..

16. A method for screening a compound which inhibits the function of the protein of claim 1 or 2, comprising:
(a) making said protein, a cancer chemotherapeutic drug and a candidate compound in contact with one another, and
(b) selecting the candidate compound as a desired candidate compound in case where the candidate compound suppresses the activity of said protein.

17. The method of claim 16, wherein the activity of said protein, whose substrate is said cancer chemotherapeutic drug, consists of a binding activity with said substrate , ATP degrading activity when said substrate binds thereto, or transporting activity of said substrate.

18. A method for screening a compound which inhibits the function of the protein of claim 1 or 2, comprising:
(a) contacting a mammalian cell that expresses the protein of claim 1 or 2 with a cancer chemotherapeutic drug and a candidate compound, and
(b) selecting the candidate compound as a desired candidate compound in case where the candidate compound enhances the toxicity of said cancer chemotherapeutic drug against said mammalian cell.

19. An inhibitor obtained by any one of methods of claims 16 to 18.

20. A method for inhibiting the function of the protein of claim 1 by using the inhibitor of claim 19.

21. A method for inhibiting the function of the protein of claim 1 by using the antibody of claim 11 or 12.

22. A method for inhibiting the function of the protein of claim 1 by using the antisense nucleotide of claim 13.

23. A method for improving the sensitivity of a patient to a cancer chemotherapeutic drug, said method comprising any one of methods of claims 20 to 22 in order to inhibit the function of the protein of claim 1.

24. The method of claim 23, wherein said cancer chemotherapeutic drug is a compound represented by the following general formula (I): 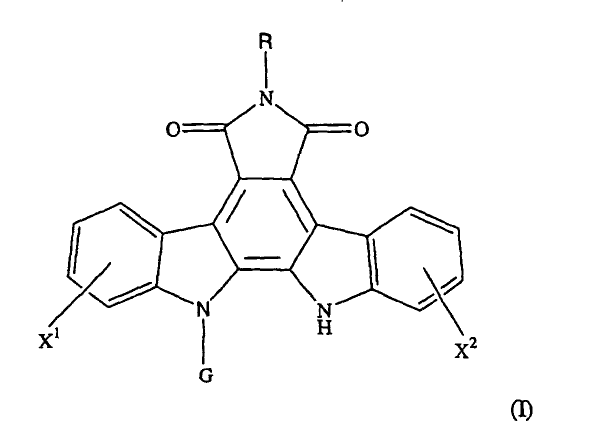 wherein X¹ and X² each independently represent a hydrogen atom, halogen atom or hydroxyl group;
R represents a hydrogen atom, amino, formylamino, or lower alkylamino which may be substituted with any one selected from the group consisting of one to three hydroxyl group(s), a pyridyl group optionally having substituent(s), and thienyl group optionally having substituent(s); and
G represents a pentose group, hexose group or derivative thereof which may be substituted with an amino group.


Sequence Listing_Abcg2.txt
SEQUENCE LISTING

<110> BANYU PHARMACEUTICAL CO., LTD

<120> Drug resistance gene and use thereof

<130> HL84783/DAN

<140>
<141>

<150> EP 01967724.4 (PCT/JP01/08112)
<151> 2001-09-18

<160> 17

<170> PatentIn Ver. 2.1

<210> 1
<211> 2027
<212> DNA
<213> Homo sapiens

<220>
<221> CDS
<222> (45)..(2009)
<223> human ABCG2

<400> 1
ttaagaccga gctctattaa gctgaaaaga taaaaactct ccag atg tct tcc agt 56
Met Ser Ser Ser
1

aat gtc gaa gtt ttt atc cca gtg tca caa gga aac acc aat ggc ttc 104
Asn Val Glu Val Phe Ile Pro Val Ser Gln Gly Asn Thr Asn Gly Phe
5                   10                  15                  20

ccc gcg aca gct tcc aat gac ctg aag gca ttt act gaa gga gct gtg 152
Pro Ala Thr Ala Ser Asn Asp Leu Lys Ala Phe Thr Glu Gly Ala Val
                25                  30                  35

tta agt ttt cat aac atc tgc tat cga gta aaa ctg aag agt ggc ttt 200
Leu Ser Phe His Asn Ile Cys Tyr Arg Val Lys Leu Lys Ser Gly Phe
            40                  45                  50

cta cct tgt cga aaa cca gtt gag aaa gaa ata tta tcg aat atc aat 248
Leu Pro Cys Arg Lys Pro Val Glu Lys Glu Ile Leu Ser Asn Ile Asn
        55                  60                  65

ggg atc atg aaa cct ggt ctc aac gcc atc ctg gga ccc aca ggt gga 296
Gly Ile Met Lys Pro Gly Leu Asn Ala Ile Leu Gly Pro Thr Gly Gly
    70                  75                  80

ggc aaa tct tcg tta tta gat gtc tta gct gca agg aaa gat cca agt 344
Gly Lys Ser Ser Leu Leu Asp Val Leu Ala Ala Arg Lys Asp Pro Ser
85                  90                  95                  100

gga tta tct gga gat gtt ctg ata aat gga gca ccg cga cct gcc aat 392
Gly Leu Ser Gly Asp Val Leu Ile Asn Gly Ala Pro Arg Pro Ala Asn
                105                 110                 115

ttc aaa tgt aat tca ggt tac gtg gta caa gat gat gtt gtg atg ggc 440
Phe Lys Cys Asn Ser Gly Tyr Val Val Gln Asp Asp Val Val Met Gly
            120                 125                 130

act ctg acg gtg aga gaa aac tta cag ttc tca gca gct ctt cgg ctt 488
Thr Leu Thr Val Arg Glu Asn Leu Gln Phe Ser Ala Ala Leu Arg Leu
        135                 140                 145

Sequence Listing_Abcg2.txt

gca aca act atg acg aat cat gaa aaa aac gaa cgg att aac agg gtc    536
Ala Thr Thr Met Thr Asn His Glu Lys Asn Glu Arg Ile Asn Arg Val
    150                 155                 160

att caa gag tta ggt ctg gat aaa gtg gca gac tcc aag gtt gga act    584
Ile Gln Glu Leu Gly Leu Asp Lys Val Ala Asp Ser Lys Val Gly Thr
165                 170                 175                 180

cag ttt atc cgt ggt gtg tct gga gga gaa aga aaa agg act agt ata    632
Gln Phe Ile Arg Gly Val Ser Gly Gly Glu Arg Lys Arg Thr Ser Ile
                185                 190                 195

gga atg gag ctt atc act gat cct tcc atc ttg ttc ttg gat gag cct    680
Gly Met Glu Leu Ile Thr Asp Pro Ser Ile Leu Phe Leu Asp Glu Pro
            200                 205                 210

aca act ggc tta gac tca agc aca gca aat gct gtc ctt ttg ctc ctg    728
Thr Thr Gly Leu Asp Ser Ser Thr Ala Asn Ala Val Leu Leu Leu Leu
        215                 220                 225

aaa agg atg tct aag cag gga cga aca atc atc ttc tcc att cat cag    776
Lys Arg Met Ser Lys Gln Gly Arg Thr Ile Ile Phe Ser Ile His Gln
    230                 235                 240

cct cga tat tcc atc ttc aag ttg ttt gat agc ctc acc tta ttg gcc    824
Pro Arg Tyr Ser Ile Phe Lys Leu Phe Asp Ser Leu Thr Leu Leu Ala
245                 250                 255                 260

tca gga aga ctt atg ttc cac ggg cct gct cag gag gcc ttg gga tac    872
Ser Gly Arg Leu Met Phe His Gly Pro Ala Gln Glu Ala Leu Gly Tyr
                265                 270                 275

ttt gaa tca gct ggt tat cac tgt gag gcc tat aat aac cct gca gac    920
Phe Glu Ser Ala Gly Tyr His Cys Glu Ala Tyr Asn Asn Pro Ala Asp
            280                 285                 290

ttc ttc ttg gac atc att aat gga gat tcc act gct gtg gca tta aac    968
Phe Phe Leu Asp Ile Ile Asn Gly Asp Ser Thr Ala Val Ala Leu Asn
        295                 300                 305

aga gaa gaa gac ttt aaa gcc aca gag atc ata gag cct tcc aag cag    1016
Arg Glu Glu Asp Phe Lys Ala Thr Glu Ile Ile Glu Pro Ser Lys Gln
    310                 315                 320

gat aag cca ctc ata gaa aaa tta gcg gag att tat gtc aac tcc tcc    1064
Asp Lys Pro Leu Ile Glu Lys Leu Ala Glu Ile Tyr Val Asn Ser Ser
325                 330                 335                 340

ttc tac aaa gag aca aaa gct gaa tta cat caa ctt tcc ggg ggt gag    1112
Phe Tyr Lys Glu Thr Lys Ala Glu Leu His Gln Leu Ser Gly Gly Glu
                345                 350                 355

aag aag aag aag atc aca gtc ttc aag gag atc agc tac acc acc tcc    1160
Lys Lys Lys Lys Ile Thr Val Phe Lys Glu Ile Ser Tyr Thr Thr Ser
            360                 365                 370

ttc tgt cat caa ctc aga tgg gtt tcc aag cgt tca ttc aaa aac ttg    1208
Phe Cys His Gln Leu Arg Trp Val Ser Lys Arg Ser Phe Lys Asn Leu
        375                 380                 385

ctg ggt aat ccc cag gcc tct ata gct cag atc att gtc aca gtc gta    1256
Leu Gly Asn Pro Gln Ala Ser Ile Ala Gln Ile Ile Val Thr Val Val
    390                 395                 400

ctg gga ctg gtt ata ggt gcc att tac ttt ggg cta aaa aat gat tct    1304
Leu Gly Leu Val Ile Gly Ala Ile Tyr Phe Gly Leu Lys Asn Asp Ser
405                 410                 415                 420

Sequence Listing_Abcg2.txt

act gga atc cag aac aga gct ggg gtt ctc ttc ttc ctg acg acc aac     1352
Thr Gly Ile Gln Asn Arg Ala Gly Val Leu Phe Phe Leu Thr Thr Asn
                425                 430                 435

cag tgt ttc agc agt gtt tca gcc gtg gaa ctc ttt gtg gta gag aag     1400
Gln Cys Phe Ser Ser Val Ser Ala Val Glu Leu Phe Val Val Glu Lys
            440                 445                 450

aag ctc ttc ata cat gaa tac atc agc gga tac tac aga gtg tca tct     1448
Lys Leu Phe Ile His Glu Tyr Ile Ser Gly Tyr Tyr Arg Val Ser Ser
        455                 460                 465

tat ttc ctt gga aaa ctg tta tct gat tta tta ccc atg agg atg tta     1496
Tyr Phe Leu Gly Lys Leu Leu Ser Asp Leu Leu Pro Met Arg Met Leu
    470                 475                 480

cca agt att ata ttt acc tgt ata gtg tac ttc atg tta gga ttg aag     1544
Pro Ser Ile Ile Phe Thr Cys Ile Val Tyr Phe Met Leu Gly Leu Lys
485                 490                 495                 500

cca aag gca gat gcc ttc ttc gtt atg atg ttt acc ctt atg atg gtg     1592
Pro Lys Ala Asp Ala Phe Phe Val Met Met Phe Thr Leu Met Met Val
                505                 510                 515

gct tat tca gcc agt tcc atg gca ctg gcc ata gca gca ggt cag agt     1640
Ala Tyr Ser Ala Ser Ser Met Ala Leu Ala Ile Ala Ala Gly Gln Ser
            520                 525                 530

gtg gtt tct gta gca aca ctt ctc atg acc atc tgt ttt gtg ttt atg     1688
Val Val Ser Val Ala Thr Leu Leu Met Thr Ile Cys Phe Val Phe Met
        535                 540                 545

atg att ttt tca ggt ctg ttg gtc aat ctc aca acc att gca tct tgg     1736
Met Ile Phe Ser Gly Leu Leu Val Asn Leu Thr Thr Ile Ala Ser Trp
    550                 555                 560

ctg tca tgg ctt cag tac ttc agc att cca cga tat gga ttt acg gct     1784
Leu Ser Trp Leu Gln Tyr Phe Ser Ile Pro Arg Tyr Gly Phe Thr Ala
565                 570                 575                 580

ttg cag cat aat gaa ttt ttg gga caa aac ttc tgc cca gga ctc aat     1832
Leu Gln His Asn Glu Phe Leu Gly Gln Asn Phe Cys Pro Gly Leu Asn
                585                 590                 595

gca aca gga aac aat cct tgt aac tat gca aca tgt act ggc gaa gaa     1880
Ala Thr Gly Asn Asn Pro Cys Asn Tyr Ala Thr Cys Thr Gly Glu Glu
            600                 605                 610

tat ttg gta aag cag ggc atc gat ctc tca ccc tgg ggc ttg tgg aag     1928
Tyr Leu Val Lys Gln Gly Ile Asp Leu Ser Pro Trp Gly Leu Trp Lys
        615                 620                 625

aat cac gtg gcc ttg gct tgt atg att gtt att ttc ctc aca att gcc     1976
Asn His Val Ala Leu Ala Cys Met Ile Val Ile Phe Leu Thr Ile Ala
    630                 635                 640

tac ctg aaa ttg tta ttt ctt aaa aaa tat tct taaatttccc cttaattc     2027
Tyr Leu Lys Leu Leu Phe Leu Lys Lys Tyr Ser
645                 650                 655

<210> 2
<211> 655
<212> PRT
<213> Homo sapiens

<400> 2

Sequence Listing_Abcg2.txt
Met Ser Ser Ser Asn Val Glu Val Phe Ile Pro Val Ser Gln Gly Asn
1               5                   10                  15

Thr Asn Gly Phe Pro Ala Thr Ala Ser Asn Asp Leu Lys Ala Phe Thr
            20                  25                  30

Glu Gly Ala Val Leu Ser Phe His Asn Ile Cys Tyr Arg Val Lys Leu
        35                  40                  45

Lys Ser Gly Phe Leu Pro Cys Arg Lys Pro Val Glu Lys Glu Ile Leu
    50                  55                  60

Ser Asn Ile Asn Gly Ile Met Lys Pro Gly Leu Asn Ala Ile Leu Gly
65                  70                  75                  80

Pro Thr Gly Gly Gly Lys Ser Ser Leu Leu Asp Val Leu Ala Ala Arg
                85                  90                  95

Lys Asp Pro Ser Gly Leu Ser Gly Asp Val Leu Ile Asn Gly Ala Pro
            100                 105                 110

Arg Pro Ala Asn Phe Lys Cys Asn Ser Gly Tyr Val Val Gln Asp Asp
        115                 120                 125

Val Val Met Gly Thr Leu Thr Val Arg Glu Asn Leu Gln Phe Ser Ala
    130                 135                 140

Ala Leu Arg Leu Ala Thr Thr Met Thr Asn His Glu Lys Asn Glu Arg
145                 150                 155                 160

Ile Asn Arg Val Ile Gln Glu Leu Gly Leu Asp Lys Val Ala Asp Ser
                165                 170                 175

Lys Val Gly Thr Gln Phe Ile Arg Gly Val Ser Gly Gly Glu Arg Lys
            180                 185                 190

Arg Thr Ser Ile Gly Met Glu Leu Ile Thr Asp Pro Ser Ile Leu Phe
        195                 200                 205

Leu Asp Glu Pro Thr Thr Gly Leu Asp Ser Ser Thr Ala Asn Ala Val
    210                 215                 220

Leu Leu Leu Leu Lys Arg Met Ser Lys Gln Gly Arg Thr Ile Ile Phe
225                 230                 235                 240

Ser Ile His Gln Pro Arg Tyr Ser Ile Phe Lys Leu Phe Asp Ser Leu
                245                 250                 255

Thr Leu Leu Ala Ser Gly Arg Leu Met Phe His Gly Pro Ala Gln Glu
            260                 265                 270

Ala Leu Gly Tyr Phe Glu Ser Ala Gly Tyr His Cys Glu Ala Tyr Asn
        275                 280                 285

Asn Pro Ala Asp Phe Phe Leu Asp Ile Ile Asn Gly Asp Ser Thr Ala
    290                 295                 300

Val Ala Leu Asn Arg Glu Glu Asp Phe Lys Ala Thr Glu Ile Ile Glu
305                 310                 315                 320

Pro Ser Lys Gln Asp Lys Pro Leu Ile Glu Lys Leu Ala Glu Ile Tyr
                325                 330                 335

Val Asn Ser Ser Phe Tyr Lys Glu Thr Lys Ala Glu Leu His Gln Leu
            340                 345                 350

Ser Gly Gly Glu Lys Lys Lys Lys Ile Thr Val Phe Lys Glu Ile Ser
        355                 360                 365

Sequence Listing_Abcg2.txt

Tyr Thr Thr Ser Phe Cys His Gln Leu Arg Trp Val Ser Lys Arg Ser
    370                 375                 380

Phe Lys Asn Leu Leu Gly Asn Pro Gln Ala Ser Ile Ala Gln Ile Ile
385                 390                 395                 400

Val Thr Val Val Leu Gly Leu Val Ile Gly Ala Ile Tyr Phe Gly Leu
                405                 410                 415

Lys Asn Asp Ser Thr Gly Ile Gln Asn Arg Ala Gly Val Leu Phe Phe
            420                 425                 430

Leu Thr Thr Asn Gln Cys Phe Ser Ser Val Ser Ala Val Glu Leu Phe
        435                 440                 445

Val Val Glu Lys Lys Leu Phe Ile His Glu Tyr Ile Ser Gly Tyr Tyr
    450                 455                 460

Arg Val Ser Ser Tyr Phe Leu Gly Lys Leu Leu Ser Asp Leu Leu Pro
465                 470                 475                 480

Met Arg Met Leu Pro Ser Ile Ile Phe Thr Cys Ile Val Tyr Phe Met
                485                 490                 495

Leu Gly Leu Lys Pro Lys Ala Asp Ala Phe Phe Val Met Met Phe Thr
            500                 505                 510

Leu Met Met Val Ala Tyr Ser Ala Ser Ser Met Ala Leu Ala Ile Ala
        515                 520                 525

Ala Gly Gln Ser Val Val Ser Val Ala Thr Leu Leu Met Thr Ile Cys
    530                 535                 540

Phe Val Phe Met Met Ile Phe Ser Gly Leu Leu Val Asn Leu Thr Thr
545                 550                 555                 560

Ile Ala Ser Trp Leu Ser Trp Leu Gln Tyr Phe Ser Ile Pro Arg Tyr
                565                 570                 575

Gly Phe Thr Ala Leu Gln His Asn Glu Phe Leu Gly Gln Asn Phe Cys
            580                 585                 590

Pro Gly Leu Asn Ala Thr Gly Asn Asn Pro Cys Asn Tyr Ala Thr Cys
        595                 600                 605

Thr Gly Glu Glu Tyr Leu Val Lys Gln Gly Ile Asp Leu Ser Pro Trp
    610                 615                 620

Gly Leu Trp Lys Asn His Val Ala Leu Ala Cys Met Ile Val Ile Phe
625                 630                 635                 640

Leu Thr Ile Ala Tyr Leu Lys Leu Leu Phe Leu Lys Lys Tyr Ser
                645                 650                 655


<210> 3
<211> 63
<212> DNA
<213> Artificial Sequence

<220>
<223> Description of Artificial Sequence:artificially
      synthesized primer sequence

<400> 3
ggccagtgaa ttgtaatacg actcactata gggaggcggt tttttttttt tttttttttt 60

Sequence Listing_Abcg2.txt
ttt                                                                    63


<210> 4
<211> 26
<212> DNA
<213> Artificial Sequence

<220>
<223> Description of Artificial Sequence:artificially
      synthesized primer sequence

<400> 4
ctcatttaaa aacttgctcg ggaacc                                           26


<210> 5
<211> 26
<212> DNA
<213> Artificial Sequence

<220>
<223> Description of Artificial Sequence: artificially
      synthesized primer sequence

<400> 5
caagaggcca gaaaagagca tcataa                                           26


<210> 6
<211> 21
<212> DNA
<213> Artificial Sequence

<220>
<223> Description of Artificial Sequence: artificially
      synthesized primer sequence

<400> 6
tactggggct tattattggt g                                                21


<210> 7
<211> 24
<212> DNA
<213> Artificial Sequence

<220>
<223> Description of Artificial Sequence:artificially
      synthesized primer sequence

<400> 7
aaaagcgatt gtcatgagaa gtgt                                             24


<210> 8
<211> 30
<212> DNA
<213> Artificial Sequence

<220>
<223> Description of Artificial Sequence:artificially
      synthesized primer sequence

<400> 8
caaaaagctt aagaccgagc tctattaagc                                       30

Sequence Listing_Abcg2.txt
<210> 9
<211> 31
<212> DNA
<213> Artificial Sequence

<220>
<223> Description of Artificial Sequence:artificially
      synthesized primer sequence

<400> 9
atcctctaga ccaggtttca tgatcccatt g                                    31


<210> 10
<211> 23
<212> DNA
<213> Artificial Sequence

<220>
<223> Description of Artificial Sequence:artificially
      synthesized primer sequence

<400> 10
gaattaaggg gaaatttaag aat                                             23


<210> 11
<211> 24
<212> DNA
<213> Artificial Sequence

<220>
<223> Description of Artificial Sequence:artificially
      synthesized primer sequence

<400> 11
agagatcgat gccctgcttt acca                                            24


<210> 12
<211> 2053
<212> DNA
<213> Artificial Sequence

<220>
<223> Description of Artificial Sequence:ABCG2-482T

<220>
<221> CDS
<222> (32)..(1999)

<400> 12
ctattaagct gaaaagataa aaactctcca g atg tct tcc agt aat gtc gaa      52
                                   Met Ser Ser Ser Asn Val Glu
                                     1               5

gtt ttt atc cca gtg tca caa gga aac acc aat ggc ttc ccc gcg aca     100
Val Phe Ile Pro Val Ser Gln Gly Asn Thr Asn Gly Phe Pro Ala Thr
        10                  15                  20

gct tcc aat gac ctg aag gca ttt act gaa gga gct gtg tta agt ttt     148
Ala Ser Asn Asp Leu Lys Ala Phe Thr Glu Gly Ala Val Leu Ser Phe
    25                  30                  35

cat aac atc tgc tat cga gta aaa ctg aag agt ggc ttt cta cct tgt     196
His Asn Ile Cys Tyr Arg Val Lys Leu Lys Ser Gly Phe Leu Pro Cys
 40                  45                  50                  55

Sequence Listing_Abcg2.txt
cga aaa cca gtt gag aaa gaa ata tta tcg aat atc aat ggg atc atg     244
Arg Lys Pro Val Glu Lys Glu Ile Leu Ser Asn Ile Asn Gly Ile Met
                60                  65                  70

aaa cct ggt ctc aac gcc atc ctg gga ccc aca ggt gga ggc aaa tct     292
Lys Pro Gly Leu Asn Ala Ile Leu Gly Pro Thr Gly Gly Gly Lys Ser
            75                  80                  85

tcg tta tta gat gtc tta gct gca agg aaa gat cca agt gga tta tct     340
Ser Leu Leu Asp Val Leu Ala Ala Arg Lys Asp Pro Ser Gly Leu Ser
        90                  95                  100

gga gat gtt ctg ata aat gga gca ccg cga cct gcc aat ttc aaa tgt     388
Gly Asp Val Leu Ile Asn Gly Ala Pro Arg Pro Ala Asn Phe Lys Cys
    105                 110                 115

aat tca ggt tac gtg gta caa gat gat gtt gtg atg ggc act ctg acg     436
Asn Ser Gly Tyr Val Val Gln Asp Asp Val Val Met Gly Thr Leu Thr
120                 125                 130                 135

gtg aga gaa aac tta cag ttc tca gca gct ctt cgg ctt gca aca act     484
Val Arg Glu Asn Leu Gln Phe Ser Ala Ala Leu Arg Leu Ala Thr Thr
                140                 145                 150

atg acg aat cat gaa aaa aac gaa cgg att aac agg gtc att caa gag     532
Met Thr Asn His Glu Lys Asn Glu Arg Ile Asn Arg Val Ile Gln Glu
            155                 160                 165

tta ggt ctg gat aaa gtg gca gac tcc aag gtt gga act cag ttt atc     580
Leu Gly Leu Asp Lys Val Ala Asp Ser Lys Val Gly Thr Gln Phe Ile
        170                 175                 180

cgt ggt gtg tct gga gga gaa aga aaa agg act agt ata gga atg gag     628
Arg Gly Val Ser Gly Gly Glu Arg Lys Arg Thr Ser Ile Gly Met Glu
    185                 190                 195

ctt atc act gat cct tcc atc ttg ttc ttg gat gag cct aca act ggc     676
Leu Ile Thr Asp Pro Ser Ile Leu Phe Leu Asp Glu Pro Thr Thr Gly
200                 205                 210                 215

tta gac tca agc aca gca aat gct gtc ctt ttg ctc ctg aaa agg atg     724
Leu Asp Ser Ser Thr Ala Asn Ala Val Leu Leu Leu Leu Lys Arg Met
                220                 225                 230

tct aag cag gga cga aca atc atc ttc tcc att cat cag cct cga tat     772
Ser Lys Gln Gly Arg Thr Ile Ile Phe Ser Ile His Gln Pro Arg Tyr
            235                 240                 245

tcc atc ttc aag ttg ttt gat agc ctc acc tta ttg gcc tca gga aga     820
Ser Ile Phe Lys Leu Phe Asp Ser Leu Thr Leu Leu Ala Ser Gly Arg
        250                 255                 260

ctt atg ttc cac ggg cct gct cag gag gcc ttg gga tac ttt gaa tca     868
Leu Met Phe His Gly Pro Ala Gln Glu Ala Leu Gly Tyr Phe Glu Ser
    265                 270                 275

gct ggt tat cac tgt gag gcc tat aat aac cct gca gac ttc ttc ttg     916
Ala Gly Tyr His Cys Glu Ala Tyr Asn Asn Pro Ala Asp Phe Phe Leu
280                 285                 290                 295

gac atc att aat gga gat tcc act gct gtg gca tta aac aga gaa gaa     964
Asp Ile Ile Asn Gly Asp Ser Thr Ala Val Ala Leu Asn Arg Glu Glu
                300                 305                 310

gac ttt aaa gcc aca gag atc ata gag cct tcc aag cag gat aag cca     1012
Asp Phe Lys Ala Thr Glu Ile Ile Glu Pro Ser Lys Gln Asp Lys Pro
            315                 320                 325

Sequence Listing_Abcg2.txt
ctc ata gaa aaa tta gcg gag att tat gtc aac tcc tcc ttc tac aaa     1060
Leu Ile Glu Lys Leu Ala Glu Ile Tyr Val Asn Ser Ser Phe Tyr Lys
        330                 335                 340

gag aca aaa gct gaa tta cat caa ctt tcc ggg ggt gag aag aag aag     1108
Glu Thr Lys Ala Glu Leu His Gln Leu Ser Gly Gly Glu Lys Lys Lys
    345                 350                 355

aag atc aca gtc ttc aag gag atc agc tac acc acc tcc ttc tgt cat     1156
Lys Ile Thr Val Phe Lys Glu Ile Ser Tyr Thr Thr Ser Phe Cys His
360                 365                 370                 375

caa ctc aga tgg gtt tcc aag cgt tca ttc aaa aac ttg ctg ggt aat     1204
Gln Leu Arg Trp Val Ser Lys Arg Ser Phe Lys Asn Leu Leu Gly Asn
                380                 385                 390

ccc cag gcc tct ata gct cag atc att gtc aca gtc gta ctg gga ctg     1252
Pro Gln Ala Ser Ile Ala Gln Ile Ile Val Thr Val Val Leu Gly Leu
            395                 400                 405

gtt ata ggt gcc att tac ttt ggg cta aaa aat gat tct act gga atc     1300
Val Ile Gly Ala Ile Tyr Phe Gly Leu Lys Asn Asp Ser Thr Gly Ile
        410                 415                 420

cag aac aga gct ggg gtt ctc ttc ttc ctg acg acc aac cag tgt ttc     1348
Gln Asn Arg Ala Gly Val Leu Phe Phe Leu Thr Thr Asn Gln Cys Phe
    425                 430                 435

agc agt gtt tca gcc gtg gaa ctc ttt gtg gta gag aag aag ctc ttc     1396
Ser Ser Val Ser Ala Val Glu Leu Phe Val Val Glu Lys Lys Leu Phe
440                 445                 450                 455

ata cat gaa tac atc agc gga tac tac aga gtg tca tct tat ttc ctt     1444
Ile His Glu Tyr Ile Ser Gly Tyr Tyr Arg Val Ser Ser Tyr Phe Leu
                460                 465                 470

gga aaa ctg tta tct gat tta tta ccc atg acg atg tta cca agt att     1492
Gly Lys Leu Leu Ser Asp Leu Leu Pro Met Thr Met Leu Pro Ser Ile
            475                 480                 485

ata ttt acc tgt ata gtg tac ttc atg tta gga ttg aag cca aag gca     1540
Ile Phe Thr Cys Ile Val Tyr Phe Met Leu Gly Leu Lys Pro Lys Ala
        490                 495                 500

gat gcc ttc ttc gtt atg atg ttt acc ctt atg atg gtg gct tat tca     1588
Asp Ala Phe Phe Val Met Met Phe Thr Leu Met Met Val Ala Tyr Ser
    505                 510                 515

gcc agt tcc atg gca ctg gcc ata gca gca ggt cag agt gtg gtt tct     1636
Ala Ser Ser Met Ala Leu Ala Ile Ala Ala Gly Gln Ser Val Val Ser
520                 525                 530                 535

gta gca aca ctt ctc atg acc atc tgt ttt gtg ttt atg atg att ttt     1684
Val Ala Thr Leu Leu Met Thr Ile Cys Phe Val Phe Met Met Ile Phe
                540                 545                 550

tca ggt ctg ttg gtc aat ctc aca acc att gca tct tgg ctg tca tgg     1732
Ser Gly Leu Leu Val Asn Leu Thr Thr Ile Ala Ser Trp Leu Ser Trp
            555                 560                 565

ctt cag tac ttc agc att cca cga tat gga ttt acg gct ttg cag cat     1780
Leu Gln Tyr Phe Ser Ile Pro Arg Tyr Gly Phe Thr Ala Leu Gln His
        570                 575                 580

aat gaa ttt ttg gga caa aac ttc tgc cca gga ctc aat gca aca gga     1828
Asn Glu Phe Leu Gly Gln Asn Phe Cys Pro Gly Leu Asn Ala Thr Gly
    585                 590                 595

Sequence Listing_Abcg2.txt
aac aat cct tgt aac tat gca aca tgt act ggc gaa gaa tat ttg gta     1876
Asn Asn Pro Cys Asn Tyr Ala Thr Cys Thr Gly Glu Glu Tyr Leu Val
600                 605                 610                 615

aag cag ggc atc gat ctc tca ccc tgg ggc ttg tgg aag aat cac gtg     1924
Lys Gln Gly Ile Asp Leu Ser Pro Trp Gly Leu Trp Lys Asn His Val
                620                 625                 630

gcc ttg gct tgt atg att gtt att ttc ctc aca att gcc tac ctg aaa     1972
Ala Leu Ala Cys Met Ile Val Ile Phe Leu Thr Ile Ala Tyr Leu Lys
            635                 640                 645

ttg tta ttt ctt aaa aaa tat tct taa atttcccctt aattcaaggg           2019
Leu Leu Phe Leu Lys Lys Tyr Ser
        650                 655

caattctgca gatatccagc acagtggcgg ccgc                               2053


<210> 13
<211> 655
<212> PRT
<213> Artificial Sequence
<223> Description of Artificial Sequence:ABCG2-482T

<400> 13

  1               5                  10                  15

             20                  25                  30

         35                  40                  45

     50                  55                  60

 65                  70                  75                  80

                 85                  90                  95

            100                 105                 110

        115                 120                 125

    130                 135                 140

145                 150                 155                 160

                165                 170                 175

            180                 185                 190

        195                 200                 205

    210                 215                 220

225                 230                 235                 240

                245                 250                 255

            260                 265                 270

        275                 280                 285

    290                 295                 300

305                 310                 315                 320

                325                 330                 335

Sequence Listing_Abcg2.txt

            340                 345                 350

        355                 360                 365

    370                 375                 380

385                 390                 395                 400

                405                 410                 415

            420                 425                 430

        435                 440                 445

    450                 455                 460

465                 470                 475                 480

                485                 490                 495

            500                 505                 510

        515                 520                 525

    530                 535                 540

545                 550                 555                 560

                565                 570                 575

            580                 585                 590

        595                 600                 605

    610                 615                 620

625                 630                 635                 640

                645                 650                 655

<210> 14
<211> 23
<212> DNA
<213> Artificial Sequence

<220>
<223> Description of Artificial Sequence:Artificially
      synthesized primer sequence

<400> 14
ccatgacgat gttaccaagt att                                          23

<210> 15
<211> 23
<212> DNA
<213> Artificial Sequence

<220>
<223> Description of Artificial Sequence:Artificially
      synthesized primer sequence

<400> 15
aacatcgtca tgggtaataa atc                                          23

Sequence Listing_Abcg2.txt

<210> 16
<211> 23
<212> DNA
<213> Artificial Sequence

<220>
<223> Description of Artificial Sequence:Artificially
      synthesized primer sequence

<400> 16
cattcatcag cctcgatatt cca                                              23


<210> 17
<211> 22
<212> DNA
<213> Artificial Sequence

<220>
<223> Description of Artificial Sequence:Artificially
      synthesized primer sequence

<400> 17
accacactct gacctgctgc ta                                               22